# EUROPEAN PATENT APPLICATION

(11) **EP 4 389 767 A1**
(43) Date of publication of application: **26.06.2024**
(21) Application number: 22857771.4
(22) Date of filing: 16.08.2022
(51) Int. Cl.: C07K 16/28, C12N 15/13, C12N 15/63, C12N 5/10, A61K 39/395, A61P 35/00

(54) **MONOCLONAL ANTIBODY TARGETING SIRP? AND USE THEREOF**

(30) Priority: 17.08.2021 CN 202110941210
(71) Applicant: Hangzhou Jiuyuan Gene Engineering Co., Ltd, 310018 Hangzhou City, Zhejiang Province (CN)
(72) Inventor: HE, Lizhen, Allentown, Pennsylvania 18106 (US); YU, Pin, Hangzhou, Zhejiang 310018 (CN); XU, Feihu, Hangzhou, Zhejiang 310018 (CN); ZHOU, Liang, Hangzhou, Zhejiang 310018 (CN); GUO, Xuancheng, Hangzhou, Zhejiang 310018 (CN); PENG, Yongbo, Hangzhou, Zhejiang 310018 (CN); CHEN, Hong, Hangzhou, Zhejiang 310018 (CN); WANG, Tongying, Hanghzou, Zhejiang 310018 (CN); SUN, Handong, Hangzhou, Zhejiang 310018 (CN); LI, Chen, Hangzhou, Zhejiang 310018 (CN)
(74) Representative: ABG Intellectual Property Law, S.L.
(86) International application number: PCT/CN2022/112677
(87) International publication number: WO 2023/020459

(57) **Abstract**

The present invention relates to the field of biomedicine, specifically to a monoclonal antibody targeting SIRPα, and a preparation method and the use thereof. The SIRPα antibody or the antigen-binding portion thereof prepared by the present invention has a high affinity for SIRPα and a low affinity for SIRPγ, which differ by two orders of magnitude. The SIRPα antibody or the antigen-binding portion thereof can efficiently block the binding of CD47 to SIRPα-V1, SIRPα-V2 and SIRPα-V8, thereby promoting the activation of macrophages, particularly enhancing the regulatory antibody-dependent cellular phagocytosis (ADCP) of a target tumor cell, bridging about innate and adaptive immune responses, and promoting the synergistic anti-cancer effect of the SIRPα antibody or the antigen-binding portion thereof with a checkpoint inhibitor PD-(L)1 monoclonal antibody. The antibody can be used for treating various cancers and microbial infectious diseases.

## Description

### FIELD OF THE INVENTION

The invention pertains to the field of biopharmaceuticals, specifically to a monoclonal antibody targeting SIRPα, and a preparation method and the use thereof.

### BACKGROUND

Signal regulatory protein alpha (SIRPα), also known as CD172a, SHPS-1, PTPNS1, BIT, or P84, is a transmembrane protein that is restrictedly expressed on all myeloid cell types, including monocytes, macrophages, neutrophils, and a subset of dendritic cells (Adams et al., 1998; Seiffert et al, 1999; Veillette et al., 1998). SIRPα contains three Ig-like domains (V, C1, and C2 domains) in the extracellular region and an immunoreceptor tyrosine-based inhibitory motif (ITIM) in the intracellular region (Fujioka et al., 1996; Kharitonenkov et al, 1997; Liu et al.,2015). Upon the engagement of ligand CD47, the tyrosine residues within cytoplasmic ITIM of SIRPα are phosphorylated, resulting in the recruitment and activation of the SH2-domain-containing protein tyrosine phosphatases SHP-1 and SHP-2, conducting inhibitory signals and leading to reduced effector functions of myeloid cells, especially phagocytosis (Fujioka et al., 1996; Kharitonenkov et al., 1997; Tsai and Discher, 2008). CD47-SIRPα axis has been demonstrated to play crucial roles in several homeostatic processes such as removal of aging red blood cells, protection of hematopoietic stem cells, and pruning of neuronal synapses (Logtenberg et al., 2020). CD47-SIRPα interaction, the so-called "don't eat me" signal, is utilized by many cancer cells through upregulating CD47 levels to escape from being phagocytosed. Therefore, the development of monoclonal antibodies (mAbs) or fusion proteins targeting either CD47 or SIRPα to disrupt the "don't eat me" signal and thus augment phagocytosis has been a well-accepted concept of antitumor therapy (Chao et al., 2010; Zhao et al., 2011; Feng, et al., 2019).

CD47-SIRPα axis, as an inhibitory signaling pathway, is considered a null event upon blocking unless in the presence of an activating signal (Logtenberg et al., 2020). CD47-SIRPα engagement has been demonstrated to counteract or attenuate agonistic signals received by myeloid cells through various membrane receptors, including 1) Fc gamma receptors (FcyRs), which bind the Fc domain of mAbs, 2) the lipoprotein-related protein (LRP), which binds calreticulin (CRT), and 3) SLAMF7 self-binding (Chao et al., 2010b; Oldenborg et al., 2001; Logtenberg et al., 2020). On the other word, disrupting the CD47-SIRPα "don't eat me" signal alone is unlikely sufficient to eradicate tumor cells, whereas the existence of "eat me" signals triggered by the interaction of Fc-FcyRs, or CRT-LRP, etc. is required for effective phagocytosis. Hence, the combination of CD47 or SIRPα blockade with tumor-opsonized antibodies [defined herein as IgG1 subtype mAbs with effector functions such as antibody-dependent cellular cytotoxicity (ADCC), antibody-dependent cellular phagocytosis (ADCP), complement-dependent cytotoxicity (CDC), e.g. anti-CD20 Rituximab or anti-EGFR Cetuximab, etc.], or epigenetic regulatory drugs such as azacytidine that upregulates CRT expression is the current strategy in clinical development (Weiskopf et al., 2017; Advani et al., 2018, Feng et al., 2019, Sallman et al., 2020).

In addition, accumulating evidence suggests that the mechanisms of action (MOA) of disrupting the CD47-SIRPα signaling pathway are beyond enhancing the phagocytosis of macrophages. First, blocking CD47-SIRPα signaling facilitates neutrophil trogocytosis of target cells that are pulled in close by tumor-modulating antibodies (Matlung et al., 2018; Bouti et al., 2021; Martínez-Sanz P et al., 2021). Second, NK cells that do not normally express SIRPα can upregulate the expression of SIRPα to counteract agonistic signaling and downregulate activity upon incubation with IL-2, while blocking CD47-SIRPα signaling enhances anti-tumor cytotoxicity of NK cells (Deuse et al., 2021). More importantly, CD47-SIRPα pathway inhibition increases phagocytosis of tumor cells by antigen-presenting cells (APCs, including dendritic cells) in the tumor microenvironment (TME) in a tumor-derived DNA driven, cGAS-STING-IRF3-IFN-I axis-dependent manner (Liu et al., 2015; Xu et al., 2017). On the one hand, it upregulates the expression and release of various pro-inflammatory cytokines and chemokines, activates NK cells, promotes the polarization of macrophages to M1, and increases the infiltration of lymphocytes in tumors; on the other hand, it promotes the maturation of dendritic cells, enhances antigen presentation and activates T cells, in particular CD8-T cells, thus bridging intrinsic immunity to adaptive immunity (Liu et al., 2015; Tseng et al., 2013; Xu et al., 2017; Gauttier et al., 2020). Furthermore, synergistic effects have been observed upon concurrently blocking both CD47-SIRPα and PD-1-PD-L1 pathways (Sockolosky et al., 2016; Gauttier et al., 2020; Kuo et al., 2020). Therefore, the combination therapies of simultaneously disrupting both innate and adaptive immune-inhibitory pathways hold promise in the treatment of PD-(L)1-refractory cancers or the so-called "cold tumors".

Early efforts were predominantly focused on targeting CD47 using mAbs or SIRPα-Fc fusion proteins. Nearly 20 CD47 blockades have entered clinical development. Since CD47 is ubiquitously expressed on normal tissue cells throughout the body, this raises the potential for on-target toxicity to healthy cells and a large "antigen sink", which are two major pain points in the development of CD47 blockades. After more than a decade of potholes since the earliest CD47 antibodies began clinical trials, no CD47 blockade has yet been approved for marketing.

Targeting SIRPα is gaining more attention and investigations. Companies who have been developing CD47 blockades such as CelgeneBMS, Forty-Seven/Gilead Science, Arch Oncology, ALX Oncology, and Innovent Biologics, are also working on SIRPα mAbs lately. Blocking CD47α-SIRP inhibitory signal through SIRPα avoids treatment-related adverse events (TRAE) (e.g., severe anemia, hemagglutination, and thrombocytopenia) for CD47-targeting agents due to the restricted expression pattern of SIRPα to myeloid cells. Anti-SIRPα antibodies are described in published international patent application disclosures: e.g., No. WO2018/057669, No. WO2018/026600, No. WO2017/178653, No. WO2017/068164, No. WO2016/063233, No. WO2016/205042, No. WO2015/138600, No. WO2013/0956352, No. WO2009/091547, No. WO2009/131453 and No. WO2009/046541, etc., all of which are hereby introduced for references.

The highly homologous sequences among SIRPα family members and the allelic variants of SIRPα with polymorphisms in the ligand binding domain have been revealed. Reportedly, the overwhelmingly dominant gene polymorphic sequences in the five major populations in East Asia, South Asia, the Americas, Europe, and Africa are the V1, V2, and V8 variants (Volts et al., 2019). Subsequent analysis of additional large data verified that only two variants, V1 and V2, and no V8 variants were detected in these five populations (Treffers et al., 2018; Sim et al., 2019). Ideally, therapeutic mAbs targeting SIRPα should recognize at least the two dominant SIRPα alleles V1 and V2.

Within the SIRP family, two other members i.e., SIRPβ and SIRPγ, have been identified and characterized (Barclay et al., 2006). The amino acid sequence of the extracellular region, especially the ligand binding V domain, is highly homologous among all the three SIRP family members. Same as SIRPα, SIRPβ is also expressed on myeloid cells. However, SIRPβ does not bind CD47 and its ligand is still unknown. Functionally, SIRPβ may transduce an activating signal through forming a complex with a transmembrane protein DAP12, which bears an immunoreceptor tyrosine-based activation motif (ITAM). It has been reported that cross-linking SIRPβ on macrophages with mAb may enhance phagocytosis (Hayashi et al., 2004; Sakamoto et al., 2022). In contrast, SIRPγ is expressed on most T cells and a subset of NK cells. SIRPγ has no cytoplasmic tail and thus no signal transduction. SIRPγ binds CD47 at about one-tenth strength of affinity related to CD47-SIRPα interaction (Brooke, et al., 2004). CD47-SIRPγ interaction may enhance T cell migration (CD47 on endothelia) and responses (CD47 on APC or tumor cells) through enhancing adherence (Piccio, et al., 2005; Dehmani et al., 2021). KWAR23, a SIRPα mAb, binds SIRPα and SIRPγ unselectively, inhibiting T cell activation likely due to disrupting CD47-SIRPγ interaction (Piccio et al., 2005; Gauttier et al., 2020). Nevertheless, it can be reasonably speculated that if SIRPα blocking mAbs impair this adherence enhancement through cross-binding SIRPγ, all CD47 blocking mAbs and fusion proteins will do the same, for their consistent blockade of CD47-SIRPα or CD47-SIRPγ.

So far, there are no SIRPα mAbs have been approved for marketing worldwide, but two have reported preliminary clinical data: the BI 765063, jointly developed by OSE Immunotherapeutics and Boehringer Ingelheim Biopharmaceuticals, and the CC-95251, developed by Celgene / Bristol-Myers Squibb (BMS).

BI 765063, aka OSE-172 or HEFLB, a humanized IgG4 mAb, is highly selective to SIRPα-V1 allele (Gauttier et al., 2020; Kuo et al., 2020), and its clinical trial enrollment has to exclude patients who do not bear SIRPα V1 allele (NCT03990233). BI 765063 monotherapy in advanced solid tumors was well tolerated and no dose-limiting toxicity (DLT) was reported up to a dose of 36 mg/kg. 50 patients received at least one dose of BI 765063, with initial clinical benefit observed in 47 patients evaluable according to RECIST 1.1 and one hepatocellular carcinoma (HCC) patient experiencing a durable partial response (PR) lasting over 9 months (Champiat et al., ASCO 2021). Subsequently, BI 765063 [18 mg/kg or 24 mg/kg, administered intravenously (IV) Q3W] in combination with a PD-1 mAb was tested in 18 heavily pretreated patients and early evidence of clinical efficacy was presented (Kotecki et al., ESMO 2021). Impressively, three PR in patients with microsatellite stable (MSS) advanced endometrium or colorectal cancer were achieved. The trial is currently recruiting patients with the above diagnoses and MSS.

CC-95251 is a fully human IgG1 mAb, with removed complement-activating functional modifications, exhibiting high affinity binding to SIRPα V1-V6 variants tested. Its combination with rituximab demonstrated a manageable safety profile in patients with heavily pretreated CD20⁺ relapsed/refractory Non-Hodgkin Lymphoma (R/R NHL) and have not yet reached MTD (Strati et al., ASH 2021). In this clinical trial, patients were treated in 28-day cycles with CC-95251 at three dose levels [3 mg/kg (n=3), 10 mg/kg (n=7), and 20 mg/kg (n=7)] IV QW and rituximab 375 mg/m² until disease progression or unacceptable toxicity were observed. The most common (≥ 30%) treatment-emergent adverse events (TEAEs) of any grade or grade ≥ 3 were neutropenia [12/17 (70.6%), 10/17 (58.8%)], and infections [10/17 (58.8%), 5/17 (29.4%)]. Among the efficacy-evaluable population (n=16) according to RECIST 1.1, the best overall response (BOR) was 68.8%, and the overall response rate (ORR) was 56.3% with 25% (4/16) patients achieving a complete response (CR). The study continues to enroll patients, and CC-95251 in combination with cetuximab for advanced solid tumors is also ongoing (NCT03783403).

Therefore, a novel SIRPα antibody is still needed in this field. On the one hand, it is required to have a high affinity for SIRPα-V1 and V2, block the interaction with the ligand CD47, inhibit the "don't eat me" signal, and not bind or weakly bind to SIRPγ, thus targeting myeloid cells without affecting the T-cell immune response. On the other hand, it is required to bridge innate immunity and adaptive immunity to maximize the efficacy of the drug while ensuring safety.

References Cited:
- Adams et al. Signal-Regulatory Protein Is Selectively Expressed by Myeloid and Neuronal Cells. J Immunol 1998; 161:1853-1859.
- Seiffert et al. Signal-regulatory protein α (SIRP α ) but not SIRP β is involved in T-cell activation, binds to CD47 with high affinity, and is expressed on immature CD34+ CD38- hematopoietic cells. BLOOD 2001; 97:2741-2749.
- Veillette et al. SIRP α -CD47 Immune Checkpoint Blockade in Anticancer Therapy. TREIMM 2017; 1449:1-12.
- Fujioka et al. A Novel Membrane Glycoprotein, SHPS-1, That Binds the SH2-Domain-Containing Protein Tyrosine Phosphatase SHP-2 in Response to Mitogens and Cell Adhesion. MOLECULAR AND CELLULAR BIOLOGY 1996; 16(12):6887 - 6899.
- Kharitonenkov et al. A family of proteins that inhibit signalling through tyrosine kinase receptors. Nature 1997; 386:181-186.
- Liu X, et al. CD47 blockade triggers T cell-mediated destruction of immuno-genic tumors. Nat Med 2015; 21: 1209 - 1215.
- Tsai and Discher et al. Self inhibition of phagocytosis: The affinity of 'marker of self' CD47 for SIRP α dictates potency of inhibition but only at low expression levels. Blood Cells, Molecules, and Diseases 2010; 45: 67-74.
- Logtenberg MEW, et al. The CD47-SIRP α Immune Checkpoint. Immunity 2020; 52:742-752.
- Chao MP, Alizadeh AA, Tang C, et al. Anti-CD47 antibody synergizes with rituximab to promote phagocytosis and eradicate non-Hodgkin lymphoma. Cell 2010; 142:699-713.
- Zhao XW, et al., CD47-signal regulatory protein- α (SIRP α ) interactions form a barrier for antibody-mediated tumor cell destruction. PNAS 2011;108:18342-18347.
- Feng M., et al: Phagocytosis Checkpoints as New Targets for Cancer Immunotherapy. Nature Reviews Cancer 2019; 19:568-586 .
- Oldenborg, P.A., Gresham, H.D., and Lindberg, F.P . CD47-signal regulatory protein alpha (SIRPalpha) regulates Fcgamma and complement receptor-mediated phagocytosis. J. Exp. Med. 2001; 193:855 - 862.
- Weiskopf K et al., Cancer immunotherapy targeting the CD47/SIRP α axis. Eur J Cancer 2017; 76:100 - 109.
- Advani R., et al. CD47 Blockade by Hu5F9-G4 and Rituximab in Non-Hodgkin' s Lymphoma. New Eng J Med 2018; 379:1711-21.
- Sallman DA, Malki MA, Asch AS, et al. Tolerability and efficacy of the first-in-class anti-CD47 antibody magrolimab combined with azacitidine in MDS and AML patients: Phase Ib results. J Clin Oncol. 2020; 38:7507.
- Treffers et al. Neutrophils Kill Antibody-Opsonized Cancer Cells by Trogoptosis. Cell Reports 2018; 23: 3946-3959.
- Sim et al.,Discovery of high affinity, pan-allelic, and pan-mammalian reactive antibodies against the myeloid checkpoint receptor SIRP α . MABS 2019; 11:1036-52.
- Barclay AN. Et al. The SIRP family of receptors and immune regulation Nature Reviews Immunology 2006; 6:457-464.
- Hayashi et al. Positive Regulation of Phagocytosis by SIRP β and Its Signaling Mechanism in Macrophages. THE JOURNAL OF BIOLOGICAL CHEMISTRY 2004; 279(28):29450-29460.
- Sakamoto et al. Anticancer efficacy of monotherapy with antibodies to SIRP α /SIRP β 1 mediated by induction of antitumorigenic macrophages. PNAS 2022; 119(1).
- Brooke, et al. Human Lymphocytes Interact Directly with CD47 through a Novel Member of the Signal Regulatory Protein (SIRP) Family. J Immunol 2004; 173:2562-2570.
- Piccio, L. et al. Adhesion of human T cells to antigen-presenting cells through SIRP β 2 - CD47 interaction costimulates T-cell proliferation. Blood 2005; 105:2421 - 2427 .
- Dehmani et al. SIRP γ -CD47 Interaction Positively Regulates the Activation of Human T Cells in Situation of Chronic Stimulation. Front. Immunol 2021; 12:732530 .
- Champiat S. et al: Safety, pharmacokinetics, efficacy, and preliminary biomarker data of first-in-class BI 765063, a selective SIRP α inhibitor: Results of monotherapy dose escalation in phase 1 study in patients with advanced solid tumors. JCO 39 (suppl 15), 2021 ASCO Annual Meeting Abstract 2623.
- Kotecki N. et al. Phase I dose escalation study in patients (pts) with advanced solid tumours receiving first-in-class BI 765063, a selective signal-regulatory protein α (SIRP α ) inhibitor, in combination with ezabenlimab (BI 754091), a programmed cell death protein 1 (PD-1) inhibitor. ESMO 2021 Congress Poster #983P.
- Strati P. et al. Interim results from the first clinical study of CC-95251, an Anti-signal regulatory protein-alpha (SIRP α ) antibody, in combination with rituximab in patients with relapsed and/or refractory non-Hodgkin lymphoma (R/R NHL). Blood 138 (suppl 1), 2021 ASH Annual Meeting Abstract 2493.
- Voets et al. Functional characterization of the selective pan-allele anti-SIRP α antibody ADU-1805 that blocks the SIRP α -CD47 innate immune checkpoint. Journal for ImmunoTherapy of Cancer 2019; 7:340.
- Andrejeva et al. Novel SIRP α Antibodies That Induce Single-Agent Phagocytosis of Tumor Cells while Preserving T Cells; J Immunol 2021.

### BRIEF SUMMARY OF THE INVENTION

The invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in IIWGX₁X₂STDYX₃X₄ALKS, wherein X₁ is D, E or N, X₂ is G, A or S, X₃ is N, Q or S, X₄ is S, T or A,
c) a heavy chain variable region CDR3 comprising the amino acid sequence set forth in SEQ ID NO: 7,
d) a light chain variable region CDR1 comprising the amino acid sequence set forth in RASESVDSYGX₅X₆FM, wherein X₅ is N, Q or S, X₆ is S, T or A,
e) a light chain variable region CDR2 comprising the amino acid sequence set forth in SEQ ID NO: 9, and
f) a light chain variable region CDR3 comprising the amino acid sequence set forth in SEQ ID NO: 10.

In another embodiment, the invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 6, SEQ ID NO: 14, SEQ ID NO: 23 or SEQ ID NO: 24,
c) a heavy chain variable region CDR3 comprising the amino acid sequence set forth in SEQ ID NO: 7,
d) a light chain variable region CDR1 comprising the amino acid sequence set forth in SEQ ID NO: 8 or SEQ ID NO: 25,
e) a light chain variable region CDR2 comprising the amino acid sequence set forth in SEQ ID NO: 9, and
f) a light chain variable region CDR3 comprising the amino acid sequence set forth in SEQ ID NO: 10.

In another embodiment, the invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 6,
c) a heavy chain variable region CDR3 comprising the amino acid sequence set forth in SEQ ID NO: 7,
d) a light chain variable region CDR1 comprising the amino acid sequence set forth in SEQ ID NO: 8,
e) a light chain variable region CDR2 comprising the amino acid sequence set forth in SEQ ID NO: 9, and
f) a light chain variable region CDR3 comprising the amino acid sequence set forth in SEQ ID NO: 10.

In another embodiment, the invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 14,
c) a heavy chain variable region CDR3 comprising the amino acid sequence set forth in SEQ ID NO: 7,
d) a light chain variable region CDR1 comprising the amino acid sequence set forth in SEQ ID NO: 8,
e) a light chain variable region CDR2 comprising the amino acid sequence set forth in SEQ ID NO: 9, and
f) a light chain variable region CDR3 comprising the amino acid sequence set forth in SEQ ID NO: 10.

In another embodiment, the invention provides a monoclonal antibody targeting SIRPα or the antigen-binding portion thereof, the CDR sequences of the heavy chain variable region and the light chain variable region can be modified by conservative sequences, including one or more substitution, addition and deletion of amino acids, etc. There are no more than five additions, deletions and/or substitutions (e.g., conservative substitutions), preferably no more than three.

In another embodiment, the invention provides a monoclonal antibody targeting SIRPα or the antigen-binding portion thereof, which comprises:
a) a heavy chain variable region comprising the amino acid sequence set forth in SEQ ID NO: 3, SEQ ID NO: 16, SEQ ID NO: 17 or SEQ ID NO: 18,
b) a light chain variable region comprising the amino acid sequence set forth in SEQ ID NO: 4, SEQ ID NO: 20, SEQ ID NO: 21, or SEQ ID NO: 22.

In another embodiment, the invention provides a monoclonal antibody targeting SIRPα or the antigen-binding portion thereof, wherein the heavy chain variable region thereof has a homologous sequence of at least 90%, 91%, 92%, 93%, 94%, 95%, 96%, 97%, 98%, 99% or 100% sequence identity with the amino acid sequence selected from SEQ ID NO: 3, SEQ ID NO: 16, SEQ ID NO: 17 or SEQ ID NO: 18; the light chain variable region thereof has a homologous sequence of at least 90%, 91%, 92%, 93%, 94%, 95%, 96%, 97%, 98%, 99% or 100% sequence identity with the amino acid sequence selected from SEQ ID NO: 4, SEQ ID NO: 20, SEQ ID NO: 21 or SEQ ID NO: 22.

In another embodiment, wherein the antibody is a full-length antibody, comprising a Fc structural domain of a human or murine IgG antibody. In an aspect, wherein the human constant region Fc structural domain is selected from the group consisting of IgGl, IgG2, IgG3, IgG4. In another embodiment, wherein the human constant region Fc structural domain is IgG1, IgG2, or IgG4. In an embodiment, wherein the human constant region Fc structural domain is IgG1w or IgG1 mutant (LALA).

In another embodiment, the invention relates to an antibody or an antibody fragment thereof is a human antibody or a human antibody fragment thereof.

In another embodiment, the invention relates to an antibody fragment that is a Fab, Fab', Fab'-SH, Fv, scFv, or F(ab')2 antibody fragment.

In another embodiment, the invention relates to an antibody fragment that is a bispecific antibody.

In another embodiment, the invention provides a monoclonal antibody targeting SIRPα, which comprises:
a) a heavy chain comprising the amino acid sequence set forth in SEQ ID NO: 28, SEQ ID NO: 29, SEQ ID NO: 30 or SEQ ID NO: 31, and
b) a light chain comprising the amino acid sequence set forth in SEQ ID NO: 26 or SEQ ID NO: 27.

In a preferred embodiment, the invention provides a monoclonal antibody targeting SIRPα, which comprises:
a) a heavy chain comprising the amino acid sequence set forth in SEQ ID NO: 31, SEQ ID NO: 35, SEQ ID NO: 36 or SEQ ID NO: 37, and
b) a light chain comprising the amino acid sequence set forth in SEQ ID NO: 27.

In a particularly preferred embodiment, the invention provides a monoclonal antibody targeting SIRPα, which comprises:
a) a heavy chain comprising the amino acid sequence set forth in SEQ ID NO: 31, a light chain comprising the amino acid sequence set forth in SEQ ID NO: 27, or
b) a heavy chain comprising the amino acid sequence set forth in SEQ ID NO: 35, a light chain comprising the amino acid sequence set forth in SEQ ID NO: 27.

In another embodiment, the invention provides isolated polynucleotides encoding the monoclonal antibody targeting SIRPα or the antibody fragment thereof. The invention provides expression vectors comprising the isolated polynucleotides, and host cells comprising the expression vectors.

In another embodiment, the invention provides a pharmaceutical composition, which comprises the monoclonal antibody targeting SIRPα or the antibody fragment thereof and a pharmaceutically acceptable carrier.

In another embodiment, the invention provides a disease treatment method, which comprises administering a therapeutically effective amount of the monoclonal antibody targeting SIRPα or the antigen-binding portion thereof to a patient with a tumor or microbial infectious disease in need of treatment.

In another embodiment, the invention provides the use of the monoclonal antibody targeting SIRPα or the antigen-binding portion thereof in combination with Rituximab in the treatment of oncological diseases.

In another embodiment, the invention provides the use of the monoclonal antibody targeting SIRPα or the antigen-binding portion thereof in combination with Daratumumab in the treatment of oncological diseases.

In another embodiment, the invention provides the use of the monoclonal antibody targeting SIRPα or the antigen-binding portion thereof in combination with a PD-(L)1 antibody in the treatment of oncological diseases.

### FIGURES

FIG. **1****.** FACS detection of AD4-12 binding to SIRPα-V1, V2 (refer to Example 4)
FIG. **2****.** FACS detection of AD4-12 and other antibodies binding to SIRPγ (refer to Example 5)
FIG. **3****.** FACS detection of AD4-12 binding to human PBMC (refer to Example 6)
FIG. **4****.** ELISA assays for AD4-12 blockade of SIRPα-V1, V2 binding to CD47 (refer to Example 7)
FIG. **5****.** FACS assays for AD4-12 blockade of SIRPα-V1, V2 binding to CD47 (refer to Example 8)
FIG. **6****.** FACS identification of macrophages derived from monocyte induced differentiation (refer to Example 9)
FIG. **7****.** Pro-phagocytosis of AD4-12 in combination with rituximab and daratumumab (refer to Example 10)
FIG. **8****.** FACS identification of dendritic cells derived from monocyte induced differentiation (refer to Example 11)
FIG. **9****.** MLR evaluation of the effect of AD4-12 on T cell activation (refer to Example 12)
FIG. **10****.** FACS detection of endocytosis of AD4-12 (refer to Example 13)
FIG. **11****.** Anti-lymphoid activity of AD4-12 in combination with rituximab *in vivo* (refer to Example 14)
FIG. **12****.** Anti-lymphoid activity of AD4-12 in combination with daratumumab *in vivo* (refer to Example 15)
FIG. **13****.** FACS comparisons of AD4-12 binding to SIRPα -V1, V2, and V8 before and after humanization (refer to Example 20)
FIG. **14****.** Octet assays for the affinity of H7L4 with SIRPα -V1 and V2 (refer to Example 21)
FIG. **15****.** ELISA assays for the binding of H7L4 to SIRPα -V1, V2, V8 (refer to Example 22)
FIG. **16****.** FACS detection of H7L4 binding to SIRPα -V1, V2, V8 and SIRPβ, SIRPγ (refer to Example 23)
FIG. **17****.** FACS detection of H7L4 binding to subpopulations of human PBMC cells (refer to Example 24)
FIG. **18****.** ELISA detection of H7L4 blockade of SIRPα-V1 and V2 binding to CD47 (refer to Example 25)
FIG. **19****.** FACS detection of H7L4 blockade of SIRPα-V1, V2, and V8 binding to CD47 (refer to Example 26)
FIG. **20****.** Pro-phagocytosis of H7L4 in combination with rituximab and daratumumab (refer to Example 27)
FIG. **21****.** Macrophage phagocytosis assays for ADCP effects of H7L4 on SIRPα⁺ cells (refer to Example 28)
FIG. **22****.** FACS assays for H7L4 reduction in SIRPα⁺ cells in mice (refer to Example 29)
FIG. **23****.** MLR evaluation of H7L4 effect on T cell activation (refer to Example 30)
FIG. **24****.** Erythrocyte agglutination test (refer to Example 31)
FIG. **25****.** Cytokine detection in the supernatant of H7L4 incubated PBMC (refer to Example 32)
FIG. **26****.** Anticancer effect of H7L4 in immunocompetent mice (A20 lymphoma cell line) (refer to Example 33)
FIG. **27****.** Anticancer effect of H7L4 in immunocompetent mice (MC38 colon cancer cell line) (refer to Example 34)
FIG. **28****.** Anticancer effects of H7L4 in combination with a PD-L1 mAb in immunocompetent mice (MC38 colon cancer cell line) (refer to Example 35)
FIG. **29****.** ELISA and FACS assays for H7L4 binding to SIRPα in cynomolgus macaques (refer to Example 36)

### DETAILED DESCRIPTION OF THE INVENTION

### Definitions

Certain terms are defined to make the invention easier to understand. Other definitions will be set forth throughout the detailed description.

Unless otherwise stated, the invention is performed using conventional techniques of molecular biology (including recombinant techniques), microbiology, cell biology, biochemistry, and immunology, which are within the scope of the art and are fully explained in the technical literature and general textbooks of the field, such as Molecular Cloning: A Laboratory Manual, etc.

The term "SIRPα" refers to wild-type signal regulatory protein alpha, or an amino acid sequence of a recombinant or non-recombinant polypeptide having the amino acid sequence of wild-type signal regulatory protein alpha, or a natural or naturally occurring allelic variant of signal regulatory protein alpha. SIRPα preferably refers to wild type mammalian SIRPα, and the most common protein variants are SIRPα v1 and v2 (reference accession numbers NP_001035111, NP_542970 (P78324) and CAA71403), in addition to SIRPα v8, among others. Polymorphisms in human SIRP result in changes in surface exposed amino acids, but which do not affect binding to CD47. The amino acid sequence of the mature form of the major wild-type human SIRPα (SIRPαV1) is shown in SEQ ID NO: 2. In one embodiment, SIRPα is a SIRPα extracellular structural domain, that is, a SIRPα protein modified to remove the transmembrane and cytoarchitectural domains, and the sequence of the extracellular structural domain of wild-type SIRPαV1 is residues 1-348 of SEQ ID NO: 2. A "variant" of SIRPα is defined as an amino acid sequence of SIRPα with one or more amino acid modifications compared to wild-type SIRPα. The variant can have "conserved" modifications, including amino acid substitutions, deletions, insertions, or a combination of these, in which the substituted amino acids have similar structural or chemical properties. SIRPα variants include polypeptides having a homologous sequence of at least 70%, 75%, 80%, 81%, 82%, 83%, 84%, 85%, 86%, 87%, 88%, 89%, 90%, 90%, 91%, 92%, 93%, 94%, 95%, 96%, 97%, 98%, 99% or greater sequence identity with wild-type SIRPα or the extracellular structural domain of wild-type SIRPα.

The term "SIRPβ" refers to the SIRPβ protein (also known as signal regulatory protein beta-1, SIRP-β-1, CD172 antigen-like family member B or CD172b) from mammalian species, preferably human SIRPβ (sequence associated with reference number 000241).

The term "SIRPγ" relates to SIRPγ from mammalian species, preferably human SIRPγ. The reference sequence of the human SIRPγ protein corresponds to the sequence of accession number AAH64532, Q9P1W8, or NM 018556.

The "antibody" refers to any form of antibody that exhibits desired biological activities (e.g., inhibition of ligand binding to its receptor or inhibition of ligand-induced receptor signal transduction). Hence, "antibody" is used in its broadest sense and expressly includes, but is not limited to, monoclonal antibodies (including full-length monoclonal antibodies), polyclonal antibodies, nanobodies, and multispecific antibodies (e.g., bispecific antibodies). A complete antibody typically contains at least two full-length heavy chains and two full-length light chains, but in some cases may comprise fewer chains, for example, antibodies naturally occurring in camelids may contain only heavy chains.

As used herein, the term "binding" and "specific binding" refers to the binding of an antibody or antigen-binding portion thereof to an antigen epitope in *in vitro* assays, preferably in bioluminescence interferometry (ForteBio) employing a purified wild-type antigen. In some embodiments, the antibody or antigen-binding portion thereof is referred to as a specific binding antigen when the antibody or antigen-binding portion thereof preferably recognizes its target antigen in a complex mixture of proteins and/or macromolecules.

A "Fc" region contains two heavy chain fragments including the CH1 and CH2 structural domains of the antibody. The two heavy chain fragments are held together by two or more disulfide bonds and by the hydrophobic interaction of the CH3 structural domain. And the Fc can be selected from the Fc structural domain of human IgGl, IgG2, IgG3, or IgG4 or mutated at one or more of these sites.

The "humanized" form of a non-human (e.g., murine) antibody is a chimeric antibody containing minimal sequences derived from non-human immunoglobulins. The majority of humanized antibodies are human immunoglobulins (acceptor antibodies) in which residues in the high-variance region of the acceptor antibody are replaced with residues in the high-variance region of non-human species (donor antibody) having the desired specificity, affinity, and capacity, such as mouse, rat, rabbit, or non-human primates. In some cases, residues in the Fv framework region (FR) of the human immunoglobulin are replaced with corresponding non-human residues. In addition, the humanized antibody may contain residues that are not present in the recipient antibody or donor antibody. These modifications are performed to further improve antibody performance. Generally, the humanized antibody comprises at least one and usually almost all of two variable structural domains, wherein all or almost all of the hypervariable loop corresponds to the hypervariable loop of non-human immunoglobulins and all or almost all of the FR region is the FR region of the human immunoglobulin sequence. The humanized antibody also optionally comprises at least a portion of the immunoglobulin (usually human immunoglobulin) constant region (Fc).

An "isolated" antibody is one that has been identified and separated from its natural environmental components, which are contaminants that interfere with the diagnostic or therapeutic application of the said antibody, and may include enzymes, hormones, and other protein solutes or non-protein solutes. In some embodiments, a said antibody is purified to more than 95% purity, preferably more than 99% purity, as determined by the Lowry method. Isolated antibodies are typically prepared by at least one purification step.

An "isolated" nucleic acid molecule is one that has been identified and separated from at least one contaminating nucleic acid molecule. The isolated nucleic acid molecule is different from its naturally occurring form or environment.

The terms "host cell", "host cell line" and "host cell culture" are used interchangeably and refer to a cell into which an exogenous nucleic acid has been introduced, including the progeny of such a cell. A host cell includes a "transformant" and a "transformed cell", which includes a primary transformed cell and progeny derived therefrom, regardless of the number of generations passed on. The progeny may not be identical to the parental cell in terms of nucleic acid content, but may contain mutations. Included herein are mutant progeny that have the same function or biological activity as those screened or selected in the initially transformed cells.

As used herein, the term "vector" refers to a nucleic acid molecule that is capable of proliferating another nucleic acid attached to it. The term includes vectors that act as self-replicating nucleic acid structures as well as vectors that bind to the genome of the host cell into which they have been introduced. Some vectors are capable of directing the expression of nucleic acids operably linked thereto. Such vectors are referred to herein as "expression vectors".

The term "immune cells" includes cells that have hematopoietic origins and play a role in immune responses. Immune cells include: lymphocytes, such as B cells and T cells; natural killer cells; and myeloid cells, such as monocytes, macrophages, eosinophils, mast cells, basophils, and granulocytes.

As used herein, a sequence "variant" is a sequence that differs from the sequence shown at one or more amino acid residues but retains the biological activity of the resulting molecule.

As used herein, the term "approximately" means that the index value is within an acceptable margin of error for a specific value measured by a person of ordinary skill in the art, the said value depending in part on how it is measured or determined (i.e., the limits of the measurement system). For example, "approximately" or "comprising substantially" can mean a range of up to 20%. In addition, especially for biological systems or processes, the term can mean up to an order of magnitude or up to 5 times the value. Unless otherwise stated, when a specific value appears in this application and claims, the meaning of " approximately" or "comprising substantially" should be assumed to be within an acceptable margin of error for that specific value.

When the terms "administration" and "treatment" are used to refer to animals, humans, subjects, cells, tissues, organs, or biological fluids, they refer to the contact of an exogenous drug, therapeutic agent, diagnostic agent, or combination with an animal, human, treated person, cell, tissue, organ, or biological fluid. The terms "administration" and "treatment" may refer, for example, to therapeutic methods, pharmacokinetic methods, diagnostic methods, research methods, and experimental methods. Treatment of cells includes contact of an agent with cells and contact of an agent with a fluid, wherein said fluid is in contact with cells. "Administration" and "treatment" also mean, for example, *in vitro* and *ex vivo* treatment of cells by reagents, diagnostic agents, binding compositions, or other cells.

An "effective amount" includes an amount sufficient to ameliorate or prevent a symptom or condition of a medical disease. The effective amount also means an amount sufficient to make a diagnosis possible or to facilitate a diagnosis. The effective amount for a specific patient may vary depending on a number of factors, such as the disease to be treated, the overall health status of the patient, the method and dose of administration, and the severity of side effects. The effective amount may be the maximum dose or dosing regimen that avoids significant side effects or toxic effects.

A "pharmaceutically acceptable carrier" includes any physiologically compatible solvents, dispersion media, coatings, antibacterial and antifungal agents, isotonic and absorption delaying agents, etc. Preferably, the vehicle for the antibody-containing composition is suitable for intravenous (IV), intramuscular, subcutaneous (SC), parenteral, spinal, or epidermal administration (e.g., by injection or infusion).

"Patient" refers to a human or non-human animal (e.g., mammal).

A "cancer" or "tumor" is a collection of cells that proliferate abnormally.

"PD-(L)1 antibody" means an antibody against PD-1 or PD-L1 target, including a PD-1 antibody, a PD-L1 antibody, or a dual antibody containing a PD-1 or PD-L1 target. Aspects of the invention will be described in further detail in the following divisions.

### SIRPα antibody

The invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in IIWGX₁X₂STDYX₃X₄ALKS, wherein X₁ is D, E or N, X₂ is G, A or S, X₃ is N, Q or S, X₄ is S, T or A,
c) a heavy chain variable region CDR3 comprising the amino acid sequence set forth in SEQ ID NO: 7,
d) a light chain variable region CDR1 comprising the amino acid sequence set forth in RASESVDSYGX₅X₆FM, wherein X₅ is N, Q or S, X₆ is S, T or A,
e) a light chain variable region CDR2 comprising the amino acid sequence set forth in SEQ ID NO: 9, and
f) a light chain variable region CDR3 comprising the amino acid sequence set forth in SEQ ID NO: 10.

In another embodiment, the invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 6, SEQ ID NO: 14, SEQ ID NO: 23 or SEQ ID NO: 24,
c) a heavy chain variable region CDR3 comprising the amino acid sequence set forth in SEQ ID NO: 7,
d) a light chain variable region CDR1 comprising the amino acid sequence set forth in SEQ ID NO: 8 or SEQ ID NO: 25,
e) a light chain variable region CDR2 comprising the amino acid sequence set forth in SEQ ID NO: 9, and
f) a light chain variable region CDR3 comprising the amino acid sequence set forth in SEQ ID NO: 10.

In an embodiment, the invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 6,
c) a heavy chain variable region CDR3 comprising the amino acid sequence set forth in SEQ ID NO: 7,
d) a light chain variable region CDR1 comprising the amino acid sequence set forth in SEQ ID NO: 8,
e) a light chain variable region CDR2 comprising the amino acid sequence set forth in SEQ ID NO: 9, and
f) a light chain variable region CDR3 comprising the amino acid sequence set forth in SEQ ID NO: 10.

In an embodiment, the invention provides a monoclonal antibody targeting SIRPα or the antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 14,
c) a heavy chain variable region CDR3 comprising the amino acid sequence set forth in SEQ ID NO: 7,
d) a light chain variable region CDR1 comprising the amino acid sequence set forth in SEQ ID NO: 8,
e) a light chain variable region CDR2 comprising the amino acid sequence set forth in SEQ ID NO: 9, and
f) a light chain variable region CDR3 comprising the amino acid sequence set forth in SEQ ID NO: 10.

In another embodiment, the invention provides a monoclonal antibody targeting SIRPα or the antigen-binding portion thereof, in which the CDR sequence homology of the heavy chain variable region and the light chain variable region is at least 90%, 91%, 92%, 93%, 94%, 95%, 96%, 97%, 98%, 99% or 100%. In some preferred embodiments, an antibody having high (i.e., 90% or greater) homology to the CDR region or variable region of a monoclonal antibody targeting SIRPα is obtained by conserved sequence modification, including one or more amino acid substitutions, additions, deletions, etc. The said one or more amino acid additions, deletions, and/or substitutions (e.g., conserved substitutions) do not exceed five, preferably not more than three. The term "conserved sequence modification" refers to amino acid modifications that do not significantly affect or alter the binding characteristics of the antibody containing the amino acid sequence. Modifications can be made by standard techniques known in the art, such as site-directed mutagenesis and PCR-mediated mutagenesis of nucleic acid molecules encoding variable region sequences. Exemplarily, an antibody prepared by the invention has one deamidation site (NS) and one isomerization site (DG) located on the heavy chain CDR2 (SEQ ID NO:6) and one deamidation site (NS) located on the light chain CDR1 (SEQ ID NO:7). Thus, the sequence general formula for the heavy chain variable region CDR2 can be summarized as IIWGX₁X₂STDYX₃X₄ALKS (SEQ ID NO:41), whereby X₁ is D, E or N, X₂ is G, A or S, X₃ is N, Q or S, and X₄ is S, T or A. The general formula for the light chain variable region CDR1 can be summarized as RASESVDSYGX₅X₆FM (SEQ ID NO:42), where X₅ is N, Q, or S and X₆ is S, T, or A.

Exemplary descriptions of CDR sequence mutations for the heavy chain variable region and the light chain variable region refer to Example 18, e.g., two sites may be mutated for heavy chain CDR2, and the general formula can be summarized as IIWGDX₁STDYNX₂ALKS (SEQ ID NO:43), wherein X₁ is G or A and X₂ is S or A, which comprises an amino acid sequence consistent with that selected from SEQ ID NO: 6, SEQ ID NO: 14, SEQ ID NO: 23 or SEQ ID NO: 24. A site mutation can be carried out on the light chain CDR1, and the general formula can be summarized as RASESVDSYGX₃SFM (SEQ ID NO:44), wherein X₃ is N or S, which comprises an amino acid sequence consistent with that selected from SEQ ID NO:8 or SEQ ID NO:25. The isomerization and deamidation site found in the CDR sequence were mutated to eliminate the PTM risk (post-translational modification) and are expected to retain the structure of the CDR loop.

In another embodiment, the invention provides a monoclonal antibody targeting SIRPα or the antigen-binding portion thereof, which comprises:
a) a heavy chain variable region comprising the amino acid sequence set forth in SEQ ID NO: 3, SEQ ID NO: 16, SEQ ID NO: 17 or SEQ ID NO: 18,
b) a light chain variable region comprising the amino acid sequence set forth in SEQ ID NO: 4, SEQ ID NO: 20, SEQ ID NO: 21, or SEQ ID NO: 22.

In another embodiment, the invention provides a monoclonal antibody targeting SIRPα or the antigen-binding portion thereof, wherein the heavy chain variable region thereof has a homologous sequence of at least 90%, 91%, 92%, 93%, 94%, 95%, 96%, 97%, 98%, 99% or 100% sequence identity with the amino acid sequence selected from SEQ ID NO: 3, SEQ ID NO: 16, SEQ ID NO: 17 or SEQ ID NO: 18; the light chain variable region thereof has a homologous sequence of at least 90%, 91%, 92%, 93%, 94%, 95%, 96%, 97%, 98%, 99% or 100% sequence identity with the amino acid sequence selected from SEQ ID NO: 4, SEQ ID NO: 20, SEQ ID NO: 21 or SEQ ID NO: 22.

Antibodies with high (i.e., 90% or greater) homology between the heavy chain variable region (VH) and light chain variable region (VL) to the VH and VL regions of the above sequences were obtained by conserved sequence modifications, including amino acid substitutions, additions, and deletions, etc. The term "conserved sequence modification" refers to an amino acid modification that does not significantly affect or alter the binding characteristics of an antibody comprising that amino acid sequence. Modifications can be made by standard techniques known in the art, such as site-directed mutagenesis and PCR-mediated mutagenesis of nucleic acid molecules encoding variable region sequences. A conserved amino acid substitution refers to the replacement of an amino acid residue with an amino acid residue having a similar side chain. Families of amino acid residues with similar side chains have been described in detail in the art. These families include amino acids with alkaline side chains (e.g., lysine, arginine, histidine), acidic side chains (e.g., aspartic acid, glutamic acid), non-charged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branching side chains (e.g., threonine, valine, isoleucine), and aromatic side chains (e.g., tyrosine, phenylalanine, tryptophan, histidine) of amino acids. Thus, one or more amino acid residues outside the CDR regions of an antibody of the invention can be replaced with other amino acid residues from the same side chain family, and the altered antibody tested for retained function using the functional assays described herein. Preferred sites for site-directed mutagenesis or PCR-mediated mutagenesis are located at sites outside the variable regions CDR1-CDR3.

### Examplary substitutions

| Original residue | Examplary substitutions | Conservative substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; | Leu |

Amino acids can be grouped according to the characteristics of common side chains:
1) Hydrophobicity: Norleucine, Met, Ala, Val, Leu, Ile;
2) Neutral hydrophilic: Cys, Ser, Thr, Asn, Gln;
3) Acidic: Asp, Glu;
4) Alkaline: His, Lys, Arg;
5) Residues affecting chain orientation: Gly, Pro;
6) Aromatic: Trp, Tyr, Phe.

Non-conserved substitution requires the exchange of a member of one of these categories for another.

In the invention, the mouse-derived antibody AD4-12 contains sequences of the heavy and light chain variable regions as shown in SEQ ID NO: 3 and SEQ ID NO: 4, respectively. The AD4-12 sequence was compared with the human germline sequence library IMGT (Lefranc, 2003), and the human germline sequence with the least amino acid differences at the position corresponding to the antibody AD4-12 frame was selected as the humanization template, and the humanization modification and revertant mutation of AD4-12 was performed with reference to Example 16, and the optimized humanized variant was basically consistent with the mouse-derived antibody AD4-12 with the same affinity (refer to Example 17), where the heavy and light chain variable region sequences of the preferred humanized antibody are:
1) a heavy chain variable region comprising the sequence of SEQ ID NO: 16, and a light chain variable region comprising the sequence of SEQ ID NO: 20,
2) a heavy chain variable region comprising the sequence of SEQ ID NO: 16, and a light chain variable region comprising the sequence of SEQ ID NO: 22,
3) a heavy chain variable region comprising the sequence of SEQ ID NO: 17, and a light chain variable region comprising the sequence of SEQ ID NO: 20,
4) a heavy chain variable region comprising the sequence of SEQ ID NO: 17, and a light chain variable region comprising the sequence of SEQ ID NO: 21,
5) a heavy chain variable region comprising the sequence of SEQ ID NO: 17, and a light chain variable region comprising the sequence of SEQ ID NO: 22,
6) a heavy chain variable region comprising the sequence of SEQ ID NO: 18, and a light chain variable region comprising the sequence of SEQ ID NO: 20,
7) a heavy chain variable region comprising the sequence of SEQ ID NO: 18, and a light chain variable region comprising the sequence of SEQ ID NO: 21,
8) a heavy chain variable region comprising the sequence of SEQ ID NO: 18, and a light chain variable region comprising the sequence of SEQ ID NO: 22.

In another embodiment, wherein the antibody is a full-length antibody containing a constant region of human IgG In yet other embodiments, wherein the antibody is a full-length antibody containing a mouse IgG constant region. A full-length heavy chain gene can be formed by linking a gene sequence encoding a heavy chain variable region to a gene sequence encoding a heavy chain constant region (CH1, CH2, and CH3) of a human antibody, and a gene sequence encoding a light chain variable region to a gene sequence encoding a light chain constant region of a human antibody to a full-length light chain gene. The sequences of the human heavy chain constant region gene and light chain constant region are known in the art (see for example Kabat, E.A. et al. (1991), Sequences of Proteins of Immunological Interest, Fifth Edition, U.S.Department of Health and Human Services, NIH Publication No.91-3242). The heavy chain constant region can be a human IgG1, IgG2, IgG3, IgG4 constant region, in another embodiment, wherein said human constant region Fc structural domain is IgG1, IgG2, or IgG4. In another embodiment, wherein said human constant region Fc structural domain is IgG1w or IgG1 mutant (LALA). The light chain constant region can be a κ or λ constant region, but most preferably a κ constant region. A full-length antibody can be expressed by inserting the antibody light chain gene and the antibody heavy chain gene into different vectors or, more commonly, inserting both genes into the same expression vector and transfecting the expression vector encoding the heavy chain and the light chain into the host cell by standard techniques.

In another embodiment, an antibody or the antibody fragment of the invention is a human antibody or the human antibody fragment.

In another embodiment, an antibody fragment of the invention is a Fab, Fab', Fab'-SH, Fv, scFv, or F(ab')2 antibody fragment.

In another embodiment, an antibody fragment of the invention is a bispecific antibody.

In another embodiment, the invention provides a monoclonal antibody targeting SIRPα, which comprises:
a) a heavy chain comprising the amino acid sequence set forth in SEQ ID NO: 28, SEQ ID NO: 29, SEQ ID NO: 30 or SEQ ID NO: 31, and
b) a light chain comprising the amino acid sequence set forth in SEQ ID NO: 26 or SEQ ID NO: 27.

In a preferred embodiment, the invention provides an isolated monoclonal antibody, wherein the heavy chain (HC) has a homologous sequence of at least 91%, 92%, 93%, 94%, 95%, 96%, 97%, 98%, 99% or 100% sequence identity to an amino acid sequence selected from SEQ ID NO: 28, SEQ ID NO: 29, SEQ ID NO: 30 SEQ ID NO: 31, SEQ ID NO: 35, SEQ ID NO: 36 or SEQ ID NO: 37; wherein the light chain (LC) has a homologous sequence of at least 91%, 92%, 93%, 94%, 95%, 96%, 97%, 98%, 99% or 100% sequence identity to an amino acid sequence selected from SEQ ID NO: 26 or SEQ ID NO: 27. An antibody with high (i.e., 90% or higher) homology to the heavy and light chains of the above sequences can be obtained by mutagenesis (e.g., site-directed mutagenesis or PCR-mediated mutagenesis) of nucleic acid molecules encoding heavy and light chain amino acids and then testing the retained function of the encoded altered antibody using the functional assays described herein. Preferred site-directed mutagenesis or PCR-mediated mutagenesis sites are located at sites other than the heavy chain variable region CDR1-CDR3 and the light chain variable region CDR1-CDR3.

In another preferred embodiment, the invention provides a monoclonal antibody targeting SIRPα, which comprises:
a) a heavy chain comprising the amino acid sequence set forth in SEQ ID NO: 31, SEQ ID NO: 35, SEQ ID NO: 36 or SEQ ID NO: 37, and
b) a light chain comprising the amino acid sequence set forth in SEQ ID NO: 27.

In another preferred embodiment, the heavy and light chain sequences of the preferred humanized antibodies of the invention are shown in the following table:

| **ID** | **Heavy chain** | **Light chain** |
|---|---|---|
| H4L3 | SEQ ID NO:28 | SEQ ID NO:26 |
| H5L3 | SEQ ID NO:29 | SEQ ID NO:26 |
| H6L3 | SEQ ID NO:30 | SEQ ID NO:26 |
| H7L3 | SEQ ID NO:31 | SEQ ID NO:26 |
| H4L4 | SEQ ID NO:28 | SEQ ID NO:27 |
| H5L4 | SEQ ID NO:29 | SEQ ID NO:27 |
| H6L4 | SEQ ID NO:30 | SEQ ID NO:27 |
| H7L4 | SEQ ID NO:31 | SEQ ID NO:27 |
| H7L4-G1w | SEQ ID NO:35 | SEQ ID NO:27 |
| H7L4-G2 | SEQ ID NO:36 | SEQ ID NO:27 |
| H7L4-G4 | SEQ ID NO:37 | SEQ ID NO:27 |

In a particularly preferred embodiment, the invention provides a monoclonal antibody targeting SIRPα, which comprises:
a) a heavy chain comprising the amino acid sequence set forth in SEQ ID NO: 31, a light chain comprising the amino acid sequence set forth in SEQ ID NO: 27, or
b) a heavy chain comprising the amino acid sequence set forth in SEQ ID NO: 35, a light chain comprising the amino acid sequence set forth in SEQ ID NO: 27.

In a specific embodiment of the invention, the monoclonal antibody targeting SIRPα or the antigen-binding portion thereof used has the following properties:
wherein the SIRPα antibody or the antigen-binding portion thereof has a high affinity of at least 10⁻¹¹ M magnitude or higher for SIRPα V1,
wherein the SIRPα antibody or the antigen-binding portion thereof can efficiently block the binding of CD47-SIRPα V1 with an IC₅₀ value of 4.7 nM or less,
wherein the SIRPα antibody or the antigen-binding portion thereof has a high affinity for SIRPα V2 and SIRPα V8 and can block the binding of both CD47-SIRPα V2 and CD47-SIRPα V8,
wherein the SIRPα antibody or the antigen-binding portion thereof has a relatively weak affinity for SIRPγ, with an affinity of approximately 10⁻⁹ M magnitude, and wherein the affinity value of the SIRPα antibody for SIRPγ is approximately 2 orders of magnitude worse than the affinity value of the SIRPα antibody for SIRPα V1,
wherein the SIRPα antibody or the antigen-binding portion thereof does not cause erythrocyte agglutination.

In another specific embodiment of the invention, the monoclonal antibody targeting SIRPα or the antigen-binding portion thereof used can further have at least one of the following properties (in particular, at least two of the following properties, in particular, all of the following properties):
wherein the SIRPα antibody or the antigen-binding portion thereof does not inhibit (particularly *in vivo*) the proliferation and/or activation of human T cells, and/or
wherein the SIRPα antibody or the antigen-binding portion thereof promotes macrophage activation, particularly the phagocytosis of tumor cells by macrophages, by blocking the SIRPα-CD47 signaling pathway, and/or
wherein said the SIRPα antibody or the antigen-binding portion thereof in combination with Rituximab has a significant promotion of Macrophage phagocytosis of tumor cells; in combination with Daratumumab also has a substantial increase in Macrophage phagocytosis; in combination with a PD-(L)1 monoclonal antibody has a synergistic anti-tumor effect.

### Nucleic acid molecules encoding antibodies of the invention

The invention also provides an isolated polynucleotide encoding a said monoclonal antibody targeting SIRPα or the antigen-binding portion thereof. The nucleic acid molecule of the invention can be DNA or RNA, and can contain or not contain an intronic sequence. In a preferred embodiment, the nucleic acid is a cDNA molecule.

The nucleic acid molecules of the invention can be obtained using standard molecular biology techniques. For antibodies expressed from hybridomas, cDNAs encoding the light and heavy chains of the antibodies prepared from hybridomas can be obtained by standard PCR amplification or cDNA cloning techniques. For antibodies obtained from immunoglobulin gene libraries (e.g., using phage display techniques), the nucleic acids encoding the antibodies can be recovered from the library.

Preferred nucleic acid molecules of the invention are those encoding amino acid sequences of the CDR region, variable region, or full-length antibody of the monoclonal antibody targeting SIRPα indicated in the invention. The DNA fragments encoding the VH and VL regions of the monoclonal antibodies targeting SIRPα described herein are further manipulated by standard recombinant DNA techniques, such as transforming the variable region gene into a full-length antibody gene, a Fab fragment gene, or a scFv gene. In these manipulations, the DNA fragment encoding VL or VH is operably linked to another DNA fragment encoding another protein, such as an antibody constant region or a flexible junction. The term "operably linked" as used herein is intended to denote linking two DNA fragments such that the amino acid sequences encoded by the two DNA fragments remain in the same reading frame.

The isolated DNA encoding the VH region can be transformed into a full-length heavy chain gene by operably linking the DNA encoding VH to another DNA molecule encoding the heavy chain constant region (CH1, CH2, and CH3). The sequence of the human heavy chain constant region gene is known in the art. The heavy chain constant region can be IgG1, IgG2, IgG3, IgG4 constant region, preferably IgG1 or IgG4 constant region. To obtain the Fab fragment heavy chain gene, the DNA encoding VH can be operably linked to another DNA molecule encoding only the heavy chain CH1 constant region.

An isolated DNA encoding the VL region can be transformed into a full-length light chain gene (and a Fab light chain gene) by operably linking the DNA encoding the VL to another DNA molecule encoding the light chain constant region CL. The sequence of the human light chain constant region gene is known in the art, and the light chain constant region can be a κ or λ constant region, but most preferably a κ constant region.

To create the scFv gene, a DNA fragment encoding VH and VL is operably linked to another fragment encoding a flexible junction, such as the amino acid sequence (Gly4-Ser)3, such that the VH and VL sequences can be expressed as adjacent single-stranded proteins, wherein the VL and VH regions are linked by a flexible junction, and the sequence general formula can be for as (G₄S)n, (SG₄)n, G₄(SG₄)n, or (G₄S)nG, etc., where n is an integer from 1-6, preferably 3-5.

### Expression vector and host cell

The invention provides an expression vector comprising a said isolated polynucleotide, and a host cell comprising the said expression vector.

As used herein, the term "vector" refers, when used herein, to a nucleic acid molecule which is capable of proliferating another nucleic acid attached to it. The vector includes plasmids, viruses, cosmids, and artificial chromosomes. Typically, an engineered vector contains replication sites, polyclonal sites, and selection markers. The vector can also include features other than transgene inserts and backbones: promoters, genetic markers, antibiotic resistance, reporter genes, targeting sequences, and protein purification tags. A vector called an expression vector (expression construct) is specific for expressing a transgene in a target cell and typically has a control sequence.

A light chain gene for the antibody and a heavy chain gene for the antibody can be inserted into different vectors or, more commonly, both genes can be inserted into the same expression vector. Antibody genes are inserted into the expression vector by standard methods. The light and heavy chain variable regions of antibodies described herein can be used to create full-length antibody genes of any antibody isotype by inserting them into an expression vector that already encodes the heavy chain constant region and light chain constant region of the desired isotype, thereby making the VH region operably linked to the CH region in the vector and the VK region operably linked to the CL region in the vector. Alternatively, the recombinant expression vector can encode a signal peptide, which facilitates the secretion of antibody chains in host cells. Antibody chain genes can be cloned into vectors so that the signal peptide is linked to the amino terminus of the antibody chain gene in the same reading frame. The signal peptide can be an immunoglobulin signal peptide or a heterologous signal peptide (i.e., a signal peptide derived from a non-immunoglobulin).

Expression vectors encoding heavy and heavy chains are transfected into host cells by standard techniques for the expression of light and heavy chains. The term "transfection" in various forms is intended to cover a variety of techniques commonly used to introduce exogenous DNA into prokaryotic or eukaryotic host cells, such as electroporation, calcium phosphate precipitation, DEAE-dextrose transfection, etc. While it is theoretically possible to express the antibodies of the invention in either prokaryotic or eukaryotic host cells, it is most preferable to express the antibodies in eukaryotic cells (most preferably in mammalian host cells). Preferred mammalian host cells for expressing recombinant antibodies of the invention include Chinese hamster ovary cells (CHO cells), NSO myeloma cells, COS cells SP2 cells, etc., preferably CHO cells.

When a recombinant expression vector encoding an antibody gene is introduced into mammalian host cells, the antibody is produced by culturing the host cells for a period of time sufficient to allow antibody expression in the host cells or, more preferably, by secreting the antibody into the medium in which the host cells are cultured. Antibodies can be recovered from the culture broth of the culture using standard protein purification methods.

### Pharmaceutical compositions

The invention provides pharmaceutical compositions comprising a said monoclonal antibody targeting SIRPα or the antibody fragment thereof and a pharmaceutically acceptable carrier.

On the one hand, the invention provides a pharmaceutical composition comprising one or a set of monoclonal antibodies targeting SIRPα or the antigen-binding portion thereof of the invention, formulated with a pharmaceutically acceptable carrier. Pharmacologically acceptable carriers include any carriers that are physiologically compatible with solvents, dispersion media, coatings, antibacterial and antifungal agents, isotonic and absorption delaying agents, etc. Preferably, the carrier is suitable for intravenous, intramuscular, subcutaneous, parenteral, spinal, or epidermal administration (e.g., by injection or infusion).

In another embodiment, the invention provides a pharmaceutical composition comprising a monoclonal antibody targeting SIRPα or the antigen-binding portion thereof, Rituximab, and a pharmaceutically acceptable carrier, which can be used to treat tumor-related diseases, particularly hematological oncological diseases.

In another embodiment, the invention provides a pharmaceutical composition comprising a monoclonal antibody targeting SIRPα or the antigen-binding portion thereof, Daratumumab, and a pharmaceutically acceptable carrier, which can be used to treat tumor-related diseases, particularly hematological oncological diseases.

In another embodiment, the invention provides a pharmaceutical composition comprising a monoclonal antibody targeting SIRPα or the antigen-binding portion thereof, a PD-(L)1 antibody, and a pharmaceutically acceptable carrier, which can be used to treat tumor-related diseases, particularly tumor immunotherapy. Wherein the said PD-(L)1 antibody comprises Nivolumab, Pembrolizumab, Cemiplimab-rwlc, Camrelizumab, Sintilimab, Toripalimab, Tislelizumab, Zimberelimab, Penpulimab, Serplulimab, Pucotenlimab, Atezolizumab, durvalumab, Avelumab, Envafolimab, Sugemalimab and Cadonilimab.

Pharmaceutical compositions must generally be sterile and stable under production and storage conditions. The compositions can be formulated into dosage forms such as solutions, microemulsions, liposomes, or lyophilized powder injections. Preferred routes of administration for the pharmaceutical compositions of the invention include intravenous, intramuscular, intradermal, intraperitoneal, subcutaneous, spinal cord/spinal, or other parenteral routes of administration, e.g., by injection or infusion.

The administered doses of antibodies of the invention range from approximately 0.01-100 mg/kg, more typically 0.1 mg/kg-100 mg/kg, or 0.5 mg/kg-50 mg/kg, or 1 mg/kg-25 mg/kg, or 2 mg/kg-10 mg/kg, or 5 mg/kg-10 mg/kg doses. Exemplary treatment regimens are administered weekly, biweekly, every three weeks, every four weeks, monthly, every 3 months, or every 3-6 months. The actual dose level of the active ingredient in the medicinal composition of the invention can be varied to obtain an amount of active ingredient that is effective in achieving the desired therapeutic response for a particular patient, composition, and mode of administration and is non-toxic to the patient. A "therapeutically effective amount" of SIRPα monoclonal antibody of the invention preferably results in a reduction in the severity of disease symptoms, an increase in the frequency and duration of disease-free periods, or the prevention of damage or disability caused by the disease. For example, for the treatment of tumors, a "therapeutically effective amount" preferably inhibits cell growth or tumor growth by at least about 20%, more preferably by at least about 40%, even more preferably by at least about 60%, and still more preferably by at least about 80%, relative to untreated subjects. The ability of the compound to inhibit tumor growth can be evaluated in an animal model system, where said animal model system can predict efficacy in human tumors. One of the ordinary skills in the art will be able to determine such amounts based on factors such as the size of the subject, the severity of the subject's symptoms, and the particular composition or route of administration selected.

### Uses

The invention provides methods of treating diseases, wherein said methods comprise administering a therapeutically effective amount of a monoclonal antibody that targets SIRPα or the antibody fragment thereof of the invention to a patient with a tumor or a microbial infectious disease in need of treatment.

A monoclonal antibody targeting SIRPα or the antigen-binding portion thereof of the invention has high affinity for SIRPα V1, SIRPα V2, and SIRPα V8, and can promote the activation of macrophages, especially the phagocytosis of tumor cells by macrophages through blocking the SIRPα-CD47 signaling pathway, especially in combination with rituximab or Daratumumab, which has a significant promotion of phagocytosis of tumor cells by Macrophage; the combination with a PD-(L)1 monoclonal antibody had a synergistic anti-tumor effect. It was also found that the antibodies provided in the invention do not cause erythrocyte agglutination and do not inhibit (especially *in vivo*) the proliferation and/or activation of human T cells, thus promising to reduce the unwanted side effects of this class of drugs in oncology treatment.

On the one hand, an antibody to SIRPα or a formulation thereof as described herein is administered to a subject in need of treatment. The term "subject" includes both human and non-human animals. Non-human animals include all vertebrates, such as mammals and non-mammals, such as non-human primates, sheep, dogs, mice, rats, cats, cattle, horses, chickens, amphibians, and reptiles. Preferred administration targets or individuals are mammals, such as mice, monkeys, dogs, cattle, horses, or humans, more preferably humans. Administration to a subject of a monoclonal antibody targeting SIRPα or the antibody fragment can eliminate or inhibit or interfere with the expression, activity, and/or signaling function of SIRPα mediated by ligand binding (e.g., CD47 binding). In an embodiment, the disease or condition associated with SIRPα expression is cancer. In some embodiments, the SIRPα antibody is administered to a patient having a cancer expressing SIRPα (e.g., a blood proliferative disorder of myeloid cells). In exemplary embodiments, the SIRPα antibodies are administered to a patient having a cancer expressing CD47.

In another embodiment, the invention provides the use of a monoclonal antibody targeting SIRPα or the antibody fragment thereof for the treatment of cancer or the inhibition of tumor growth. The tumor or cancer is selected from squamous cell carcinoma, small cell lung cancer, non-small cell lung cancer, squamous non-small cell lung cancer (NSCLC), non-squamous NSCLC, glioma, gastrointestinal cancer, kidney cancer, ovarian cancer, liver cancer, colorectal cancer, endometrial cancer, prostate cancer, thyroid cancer, neuroblastoma, pancreatic cancer, glioblastoma, cervical cancer, gastric cancer, bladder cancer, head and neck cancer, melanoma, bone cancer, skin cancer, diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, etc., and any combination of those cancers. The antibody targeting SIRPα of the invention can also be used to treat metastatic cancers.

In another embodiment, the invention provides the use of the said monoclonal antibody targeting SIRPα or the antigen-binding portion thereof in combination with Rituximab in the treatment of oncological diseases. In another embodiment, wherein said oncological disease is a hematological tumor. In another embodiment, wherein said hematologic oncologic disease is diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, etc.

In another embodiment, the invention provides the use of the said monoclonal antibody targeting SIRPα or the antigen-binding portion thereof in combination with Daratumumab in the treatment of oncological diseases. In another embodiment, wherein said oncological disease is a hematological tumor. In another embodiment, wherein said hematologic oncologic disease is diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, etc.

In another embodiment, the invention provides the use of the monoclonal antibody targeting SIRPα or the antigen-binding portion thereof in combination with a PD-(L)1 antibody in the treatment of oncological diseases. Wherein said a PD-(L)1 antibody comprises a PD-1 antibody, a PD-L1 antibody or a bispecific antibody against PD-1/PD-L1; wherein said a PD-1 antibody comprises Nivolumab, Pembrolizumab, Cemiplimab-rwlc, Camrelizumab, Sintilimab, Toripalimab, Tislelizumab, Zimberelimab, Penpulimab, Serplulimab, Pucotenlimab, etc. A PD-L1 monoclonal antibody comprises Atezolizumab, durvalumab, Avelumab, Envafolimab, Sugemalimab, etc. A PD-L1 bispecific antibody comprises Cadonilimab targeting PD-1 and CTLA-4. In another embodiment, wherein said oncological disease is hematological tumors, malignant melanoma, breast cancer, small cell lung cancer, non-small cell lung cancer, liver cancer, gastric cancer, kidney cancer, colorectal cancer, bladder cancer, head and neck tumors, cervical cancer, Merkel cell carcinoma, and all microsatellite highly unstable (MSI-H) solid tumor treatments, etc. In another embodiment, wherein said hematological neoplastic disease is diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, etc. In another embodiment, the invention provides the use of a monoclonal antibody targeting SIRPα or the antibody portions thereof for the treatment of microbial infectious diseases. A monoclonal antibody targeting SIRPα or the antigen-binding portion thereof of the invention promotes the activation of macrophages, thereby enabling the induction or maintenance of phagocytosis in individuals. Phagocytosis includes phagocytosis induced by professional phagocytes (e.g., monocytes, macrophages, neutrophils, dendritic cells, or mast cells), non-professional phagocytes (e.g., epithelial cells, endothelial cells, fibroblasts, or mesenchymal cells), or both. The invention provides methods for treating a viral infection or bacterially infected disease in an individual comprising administering to an individual suffering from a viral infection or bacterially infected disease a monoclonal antibody targeting SIRPα or the antigen-binding portion thereof as described herein. The viral infection or bacterial infection disease or condition is chronic or acute. Wherein said viral infectious diseases include diseases caused by infections such as adenovirus, herpes virus, papillomavirus, coronavirus, human immunodeficiency virus (HIV), human cytomegalovirus, EB virus, hepatitis C virus or hepatitis B virus; said bacterial infectious diseases include diseases caused by infections such as Bacillus, Chlamydia pneumoniae, Haemophilus influenzae, Mycobacterium tuberculosis, Pseudomonas, Salmonella, Staphylococcus, Streptococcus, and dense spirochetes.

The optimal dose of a monoclonal antibody that targets SIRPα or the antibody fragment of the invention depends on the disease under treatment, the severity of the disease, and the presence or absence of side effects. The optimal dose can be determined by routine experiments. For parenteral administration, doses of 0.1 mg/kg-100 mg/kg, or 0.5 mg/kg-50 mg/kg, or 1 mg/kg-25 mg/kg, or 2 mg/kg-10 mg/kg, or 5 mg/kg-10 mg/kg are given. Exemplary regimens can be administered weekly, biweekly, every three weeks, every four weeks, monthly, every 3 months, or every 3-6 months.

### EXAMPLES

The following Examples are provided to completely disclose and describe how to prepare, screen, characterize, and use the invention, and these Examples are not intended to limit the scope of the invention in any way, nor do they represent that the experiments described below are all or only the experiments performed. The inventor guarantees the objectivity and accuracy of the experimental data, but a certain amount of experimental error and bias should be allowed.

### Example 1: Immunization of mice to obtain mouse-derived antibody sequences

A mouse-derived monoclonal antibody against human SIRPα was prepared. BALB/C mice were first immunized with recombinant SIRPα extracellular domain Fc fusion protein SIRPα V1-Fc (SEQ ID NO: 1) purchased from ACRO (Cat# SIA-H5251), followed by CHO cells transfected with SIRPα V1 (SEQ ID NO: 2) as antigen, and the immune response of mice was monitored by ELISA. Spleen RNA of mice was extracted when the anti-human SIRPα immunoglobulin titer reached the requirement, and the VH and Yκ genes were amplified by RT-PCR and cloned into phage for display and panning, to obtain antibody fragments with high SIRPα affinity and function of blocking CD47-SIRPα. The heavy chain variable region (SEQ ID NO: 3) and light chain variable region (SEQ ID NO: 4) of AD4-12 were obtained by sequencing. Kabat numbers of mouse antibody sequences were obtained using the software ANARCI (Dunbar and Deane 2015). CDRs (TABLE 2, SEQ ID NO: 5-10) were determined according to the definitions published on the antibody website http://www.bioinf.org.uk/ (Martin, 2018).

**TABLE 1**

| Name | Amino acid sequence |
|---|---|
| SIRPα-Fc protein (SEQ ID NO: 1) | EEELQVIQPDKSVLVAAGETATLRCTATSLIPVGPIQWFRG AGPGRELIYNQKEGHFPRVTTVSDLTKRNNMDFSIRIGNI TPADAGTYYCVKFRKGSPDDVEFKSGAGTELSVRAKPSA PVVSGPAARATPQHTVSFTCESHGFSPRDITLKWFKNGNE LSDFQTNVDPVGESVSYSIHSTAKVVLTREDVHSQVICEV AHVTLQGDPLRGTANLSETIRVPPTLEVTQQPVRAENQV NVTCQVRKFYPQRLQLTWLENGNVSRTETASTVTENKD GTYNWMSWLLVNVSAHRDDVKLTCQVEHDGQPAVSKS HDLKVSAHPKEQGSNTAAENTGSNERPKSCLKTHTCPPC PAPELLGGPSVFLFPPKPKDTLMISRTPEVTCVVVDVSHE DPEVKFNWYVDGVEVHNAKTKPREEQYNSTYRVVSVLT VLHQDWLNGKEYKCKVSNKALPAPIEKTISKAKGQPREP QVYTLPPSRDELTKNQVSLTCLVKGFYPSDIAVEWESNGQ PENNYKTTPPVLDSDGSFFLYSKLTVDKSRWQQGNVFSC SVMHEALHNHYTQKSLSLSPGK |
| SIRPα full-length protein (SEQ ID NO: 2) | EEELQVIQPDKSVLVAAGETATLRCTATSLIPVGPIQWFRG AGPGRELIYNQKEGHFPRVTTVSDLTKRNNMDFSIRIGNI TPADAGTYYCVKFRKGSPDDVEFKSGAGTELSVRAKPSA PVVSGPAARATPQHTVSFTCESHGFSPRDITLKWFKNGNE LSDFQTNVDPVGESVSYSIHSTAKVVLTREDVHSQVICEV AHVTLQGDPLRGTANLSETIRVPPTLEVTQQPVRAENQV NVTCQVRKFYPQRLQLTWLENGNVSRTETASTVTENKD GTYNWMSWLLVNVSAHRDDVKLTCQVEHDGQPAVSKS HDLKVSAHPKEQGSNTAAENTGSNERNIYIVVGVVCTLL VALLMAALYLVRIRQKKAQGSTSSTRLHEPEKNAREITQ DTNDITYADLNLPKGKKPAPQAAEPNNHTEYASIQTSPQP ASEDTLTYADLDMVHLNRTPKQPAPKPEPSFSEYASVQVP RK |
| Heavy chain variable region of AD4-12 (SEQ ID NO: 3) | EVQLEESGPGLVAPSQSLSITCTVSGFSLTGYGVNWVRQP PGKGLEWLGIIWGDGSTDYNSALKSRLSISKDNSKSQVFL KMNSLQSDDTARYYCARAGKMDYWGQGTSVTVSS |
| Light chain variable region of AD4-12 (SEQ ID NO: 4) | DIVLTQSPASLAVSLGQGATISCRASESVDSYGNSFMHWY QQKPGQPPKPLIYLASNLESGVPARFSGSGSRTDFTLTIDP VEADDAATYYCQQNNEYPWTFGGGTKLEIK |

**TABLE 2. CDR sequences of AD4-12 variable region**

| | CDR | SEQ ID NO: | Sequence |
|---|---|---|---|
| Heavy chain variable region of AD4-12 | HCDR1 | 5 | GFSLTGYGVN |
| | HCDR2 | 6 | IIWGDGSTDYNSALKS |
| | HCDR3 | 7 | AGKMDY |
| Light chain variable region of AD4-12 | LCDR1 | 8 | RASESVDSYGNSFM |
| | LCDR2 | 9 | LASNLES |
| | LCDR3 | 10 | QQNNEYPWT |

### Example 2: Expression and purification of AD4-12 antibody

The cDNAs of AD4-12's VL and VH regions were screened and linked to the human κ light chain and the IgG1, IgG2, and IgG4 heavy chain constant regions, respectively, and mutant sequences were also designed to enhance or weaken the binding to the Fc receptor for the Fc segment of the IgG1 and IgG2 heavy chain constant regions, respectively. The constructed AD4-12 human chimeric light and heavy chains were cloned into the expression vector pCDNA3.1(+). The expression vector plasmid was transiently transferred into ExpiCHO-S cells for expression using the medium (Gibco, Cat# A29100-01) and the transfection kit (Gibco, Cat# A29129).

Method: ExpiCHO-S cells were passaged one day before transfection, and the constructed plasmid was added dropwise to the ExpiCHO-S cell culture in a 25 mL system after mixing with the transfection reagent and mixed thoroughly. The mixture was incubated at 37°C for 18-22 h, then placed in a shaker incubator at 32°C and 5% CO₂ after addition of supplementation medium for 5 days and added supplementation medium again. The supernatant was collected after 10-12 days, and the target antibodies of different IgG subtypes: AD4-12-G1 and AD4-12-G1ₗₐₗₐ were purified by the conventional Protein A over-column method.

The following embodiments focus on the evaluation of the IgG1 wild type (AD4-12-G1) and the mutant (AD4-12-G1ₗₐₗₐ) comprising the L234A/L235A (LALA) amino acid substitution to eliminate binding to the Fc receptor. In embodiments where AD4-12-G1 and AD4-12-G1ₗₐₗₐ do not differ both are uniformly labeled as AD4-12, and only when AD4-12-G1 and AD4-12-G1ₗₐₗₐ are compared are the two labeled separately.

### Example 3: Affinity assay of AD4-12 with SIRPα-V1, SIRPα-V2, SIRPβ, SIRPγ, and cynomolgus macaques SIRPα by Biolayer Interferometry (BLI)

The affinity of AD4-12 binding to SIRPα, SIRPβ, and SIRPγ was determined by ForteBio Octet RED 96. SIRPα V1-his (ACRO, Cat# SIA-H5225), SIRPα V2-his (made by transient transfection of HEK293F cells according to SEQ ID NO:38 sequence), SIRPβ-his (Novoprotein, Cat# CS93), SIRPγ-his (Sino Biological, Cat# 11828-H08H) or cynomolgus macaques SIRPα-his (ACRO, Cat# SIA-C52H7) at 5 µg/mL was loaded to a Ni-NTA biosensor for 600 s. AD4-12 and reference 18D5 (OSE Immunotherapeutics' SIRPα antibody BI 765063 parent mouse-derived antibody, synthetic sequence reference CN201780023581.2, Fc fragment is IgG4) were prepared at 1:2 diluted series of 7 concentrations. The starting concentration of antibody and reaction dissociation time for different cured protein molecules differ: for SIRPα V1, SIRPα V2, and SIRPβ cured sensors, starting concentration of the antibody is 25 nM, the binding time is 200 s and the dissociation time is 1000 s; for SIRPγ cured sensors, starting concentration of antibody is 200 nM, binding time is 200 s and dissociation time is 800 s; for SIRPγ cured sensors, starting concentration of antibody was 200 nM, binding time was 200 s and dissociation time was 800 s; for cynomolgus macaques SIRPα cured sensors, binding time was 200 s and dissociation time was 600 s. The data were fitted to calculate the affinity.

Results: As shown in TABLE 3-1, AD4-12 of the invention has high affinity for SIRPα-V1, which is one order of magnitude higher compared with the reference 18D5; AD4-12 has the same high affinity for SIRPα-V2; AD4-12 binds to SIRPβ with slightly weaker affinity than to SIRPα; AD4-12 also binds to SIRPγ, but affinity was two orders of magnitude lower than that of binding to SIRPα. In addition, AD4-12 was able to cross-bind cynomolgus macaque SIRPα molecules with an affinity similar to that of binding human SIRPα molecules.

**TABLE 3-1: Affinity assay for AD4-12 binding to SIRP family members by Octet**

| Sample | | Affinity KD(M) | Binding Rate kon(1/Ms) | Dissociation Rate kdis(1/s) |
|---|---|---|---|---|
| AD4-12 | SIRPα V1 | 1.11E-11 | 1.05E+06 | 1.16E-05 |
| | SIRPα V2 | 8.98E-10 | 3.64E+05 | 3.27E-04 |
| | SIRPβ | 3.53E-11 | 9.09E+05 | 3.21E-05 |
| | SIRPγ | 1.03E-09 | 7.09E+05 | 7.31E-04 |
| | Monkey SIRPα | 4.30E-10 | 1.16E+06 | 4.98E-04 |
| 18D5 | SIRPα V1 | 1.15E-10 | 6.74E+06 | 7.76E-05 |
| | SIRPα V2 | 1.07E-09 | 5.61E+05 | 6.01E-04 |
| | SIRPβ | 1 .37E-11 | 1.72E+06 | 2.36E-05 |
| | SIRPγ | 1.23E-09 | 7.28E+05 | 8.96E-04 |

As shown in TABLE 3-2, AD4-12 of the invention has a high affinity for SIRPα-V1, V2, and V8 with significantly lower EC₅₀ values than the control antibody 18D5; and AD4-12 binds weaker to SIRPβ than the control antibody 18D5.

**TABLE 3-2 Comparison of EC₅₀ values of AD4-12 binding to SIRP family members**

| EC₅₀(nM) | ELISA | | FACS | | |
|---|---|---|---|---|---|
| | SIRPαV1 | SIRPβ | SIRPαV1 | SIRPαV2 | SIRPαV8 |
| AD4-12 | 0.04885 | 0.2707 | 0.6153 | 0.2450 | 0.4073 |
| 18D5 | 0.1296 | 0.1963 | 1.077 | 3.651 | 8.759 |

### Example 4: Detection of AD4-12 binding to SIRPα-V1, V2 by flow cytometry (FACS)

Cell lines with high expression of SIRPα-V1 (SEQ ID NO: 2), V2 (SEQ ID NO: 38), or V8 (SEQ ID NO: 39) constructed by stable transfer were used to analyze the binding ability of AD4-12 to SIRPα molecules on the cell membrane by flow cytometry. Logarithmically grown 293T-SIRPα-V1 cells and CHOZN-SIRPα-V2 cells at 2×10⁵ cells/well were dispensed into 96-well U-shaped culture plates, incubated with Fc blocker (BD Pharmingen, Cat# 564219) and then the serially diluted AD4-12 or reference 18D5 at 4°C for 30 min, respectively. After washing, cells were stained with 0.2 µg goat anti-hIgG Fc-AF488 (Jackson ImmunoResearch, Cat# 109-546-170) at 4°C for 30 min. After washing again, cells were resuspended in 50 µL of DPBS/1% FBS and analyzed by FlowJo V10 and GraphPad Prism6 software.

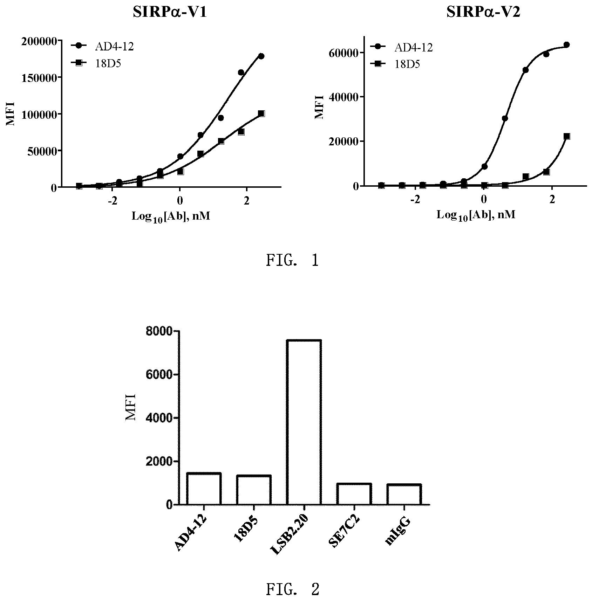

Results: As shown in FIG. 1, AD4-12 had an excellent binding activity to the stably transformed cell line expressing SIRPα-V1 protein, which was slightly better than that of 18D5; AD4-12 could bind well to the stably transformed cell line expressing SIRPα-V2 protein, which was significantly better than that of 18D5.

### Example 5: Detection of AD4-12 binding to SIRPγ by FACS

Logarithmically grown Jurkat cells at 2×10⁵ cells/well were dispensed into 96-well U-shaped culture plates, incubated with Fc blocker and then 2 µg/mL of corresponding antibodies among which LSB2.20 (SantaCruz, Cat# sc-53604) was SIRPy-specific antibody and SE7C2 (SantaCruz, Cat # sc-23863) was SIRPα-specific antibody, at 4°C for 30 min. After washing, cells were stained with 0.2 µg of goat anti-mIgG Fc-AF488 (Jackson ImmunoResearch, Cat# 115-545-003) or goat anti-hIgG Fc-AF488 at 4°C for 30 min. After washing again, cells were resuspended in 50 µL of DPBS/1% FBS and analyzed by FlowJo V10 and GraphPad Prism6 software.

Results: As shown in FIG. 2, LSB2.20 and SE7C2 verified that Jurkat cells were SIRPγ-positive and SIRPα-negative. The antibody AD4-12 did not bind to Jurkat cells essentially, similar to 18D5.

### Example 6: Binding of AD4-12 to human PBMC by FACS

5×10⁵/well of human peripheral blood mononuclear cells (PBMC), derived from 3 healthy donors (purchased from Milestone^{®} Biotechnologies, Cat# PB003F), were dispensed into 96-well U-shaped plates. After blocking with Fc blocker and mIgG (Invitrogen, Cat# 31903), cells were incubated with 3 µg of biotin-labeled AD4-12, or isotype control. Subsequently, 0.2 µg of cell surface marker antibodies [anti-CD56-APC (BD Pharmingen, Cat# 555518), anti-CD11b- eFlour 506 (eBioscience^{™}, Cat# 69-0118-42), anti-CD3-FITC (eBioscience^{™}, Cat# 17-0038-42), anti-CD14-APC eFlour 780 (eBioscience ^{™}, Cat# 47-0149-42) and anti-CD19-eFlour 450 (eBioscience^{™}, Cat# 48-0198-42)] and streptavidin-PE (BD Pharmingen, Cat# 554061) were added for staining at 4°C for 30 min in turn. After washing, cells were resuspended in DPBS/1%FBS buffer containing 0.5 µg of 7AAD (BD Pharmingen, Cat# 559925) and analyzed by FACS. Staining index = MFI (AD4-12)/MFI (isotype).

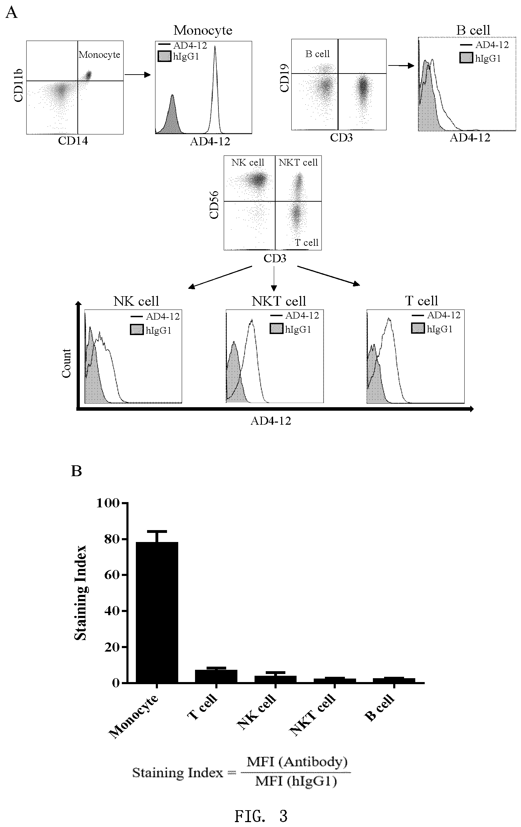

Results: As shown in FIG. 3, AD4-12 bound strongly to monocytes (CD11b⁺ CD14⁺), weakly to T cells (CD3⁺ CD56⁻), NK cells (CD3⁻ CD56⁺), and NKT cells (CD3⁺ CD56⁺), and not to B cells (CD19⁺ CD3⁻) in PBMC. (A) Representative flow cytometry analysis; (B) staining index of 3 donors. Data were expressed as mean ± SD.

### Example 7: Blockade of SIRPα-V1, V2 binding to CD47 by AD4-12 detected by enzyme-linked immunosorbent assay (ELISA)

100 µL of 2.5 µg/mL of recombinant SIRPα-V1-his and SIRPα-V2-his were coated overnight at 4°C in Maxisorp ELISA 96-well plates. Serially diluted AD4-12 and reference 18D5 were mixed with 0.25 µg of biotin-CD47 [CD47 was expressed by plasmid constructed according to Genbank Accession NP_942088 sequence as QLLFNKTKSVEFTFCNDTVVIPCFVTNMEAQNTTEVYVKWKFKGRDIYTFDGALNKS TVPTDFSSAKIEVSQLLKGDASLKMDKSDAVSHTGNYTCEVTELTREGETIIELKYRV VSWFSP (SEQ ID NO: 40)] and incubated in the microtiter plate for 1 h at 37°C. After washing, each well was added with Streptavdin-HRP (Thermo, Cat# 434323), substrate TMB (Beyotime, Cat# P0209-100mL), and read by an enzyme marker.

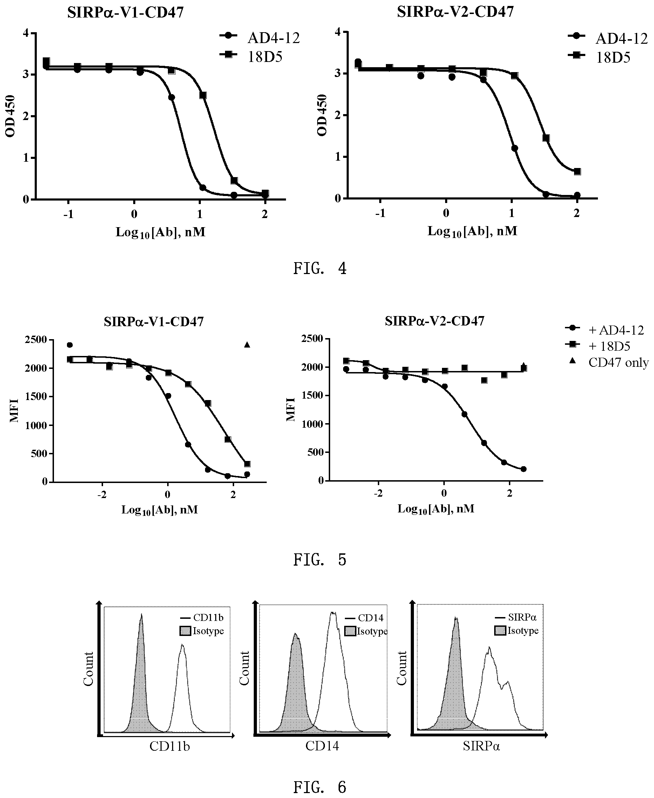

Results: As shown in FIG. 4, AD4-12 could block SIRPα-V1 and SIRPα-V2 binding to CD47 with IC₅₀ values of 5.258 nM and 9.322 nM, respectively, and the blocking effect was better than that of 18D5.

### Example 8: Blockade of SIRPα-V1, V2 binding to CD47 by AD4-12 detected by FACS

Logarithmically grown 293T-SIRPα-V1 cells and CHOZN-SIRPα-V2 cells at 2×10⁵ cells/well were dispensed into 96-well U-shaped culture plates, incubated with Fc blocker and then 0.5 µg of anti-CD47-FITC (homemade) and serially diluted AD4-12 or 18D5 at 4°C for 30 min. After washing, cells were analyzed by FlowJo V10 and GraphPad Prism6 software.

Results: As shown in FIG. 5, the interaction between SIRPα-V1-CD47 and SIRPα-V2-CD47 was blocked well by AD4-12 with IC₅₀ values of 1.742 nM and 6.417 nM, respectively, while reference 18D5 showed weaker blockade of SIRPα-V1-CD47 than AD4-12 and no blocking activity for SIRPα-V2-CD47.

### Example 9: Induced differentiation and identification of macrophages

Fresh PBMC (Milestone^{®} Biotechnologies, Cat# PB003F) were washed with 1× BD IMag^{™} buffer (BD Pharmingen, Cat# 552362) and then incubated with 50 µL of BD IMag^{™} Anti-Human CD14 Magnetic Particles-DM (BD Pharmingen, Cat# 557769) per 1×10⁷ cells for 30 minutes at room temperature. CD14⁺ monocytes were isolated by Cell Separation Magnet after washing, resuspended in IMDM medium (containing 10% FBS + 50 uM β-mercaptoethanol + 70 ng/ml M-CSF), dispensed in 96-well plates with 1×10⁵ cells per well, placed in a 5% CO₂ incubator at 37°C, and continued to be cultured for 3 days with the same medium replenished until 7 days. Macrophage phenotype was identified by flow cytometry after digestion with 0.25% Trpsin-EDTA. The cells were blocked with Fc blocker formulated in 1 mg/mL mIgG at 4°C for 15 min, and then stained with fluorescein-labeled anti-CD11b, anti-CD14, and anti-SIRPα antibodies (SantaCruz, Cat# sc-23863PE), respectively, and analyzed by FlowJo V10 and GraphPad Prism6 software.

Results: As shown in FIG. 6, CD11b⁺CD14⁺SIRPα⁺ macrophages indicated successful induction of differentiation.

### Example 10: Pro-phagocytosis of AD4-12 in combination with Rituximab and Daratumumab

1) CFSE staining of Raji cells
   1 mL of 8×10⁶ cells/mL of logarithmic growth phase Raji cells (positive for CD20, CD38, CD47) was mixed with 1 mL of DPBS containing 2 µL of 0.15 mM CFSE, incubated in 37°C for 15 min, and then added 2 mL of serum to terminate the reaction. The cells were resuspended in RPMI 1640 medium at a density of 2×10⁶/mL after washing.
2) Co-incubation of macrophages with Raji cells
   The 96-well plates with pre-induced differentiation of macrophages (see Example 9) were incubated with 10 µg/mL of AD4-12 per well for 30 min at 37°C in a 5% CO₂ incubator. CFSE-labeled Raji cells, with or without rituximab (RTX) or daratumumab (Dara) at a final concentration of 0.1 µg/mL, were incubated for 30 min at 37°C in a 5% CO₂ incubator as target cells. The CFSE-labeled Raji cells were pipetted into the 96-well plate with macrophages at a ratio of 1:5, i.e., 1×10⁵/well for macrophages and 5×10⁵/well for Raji cells, and incubated for 2 h at 37°C in a 5% CO₂ incubator.
3) Phagocytic activity detected by flow cytometry
   The mixed cells were digested with 250 µL of 0.25% Trpsin-EDTA, incubated with 1.5 µg of Fc blocker formulated in 1 mg/mL of IgG1 solution after washing and then 0.2 µg of anti-CD11b-APC for 30 min at 4°C. After washing, cells were resuspended in DPBS/1%FBS buffer containing 0.5 µg of 7AAD and analyzed by FACS. FlowJo V10 software was used to compare the proportion of CFSE staining in the CD11b positive population after the removal of dead and adherent cells.

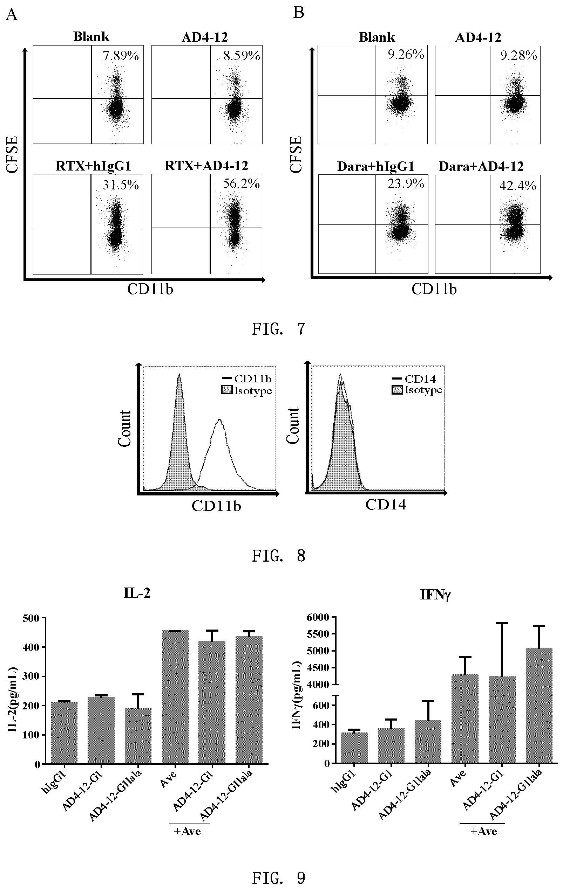

Results: As shown in FIG. 7, AD4-12 had no prophagocytic effect by itself, but showed a significant enhancement of both antibody-dependent cellular phagocytosis (ADCP) in combination with (A) rituximab or (B) daratumumab.

### Example 11: Differentiation and identification of dendritic cell induction

Fresh PBMC were incubated with 50 µL of BD IMag^{™} Anti-Human CD14 Magnetic Particles-DM (BD Pharmingen, Cat# 557769) per 1×10⁷ cells for 30 minutes at room temperature and isolated with Cell Separation Magnet to obtain CD14⁺ monocytes. The CD14⁺ monocytes were cultured in IMDM medium (containing 10% FBS + 50 uM β-mercaptoethanol + 100 ng/ml GM-CSF + 70 ng/ml IL-4) at a density of 1×10⁵ cells per bottle in a 5% CO₂ incubator at 37°C for 3 days and continued to be cultured until 7 days after supplemented the same medium. After verifying the phenotype, the cells were incubated with Fc blocker formulated in 1 mg/mL of IgG1 solution for 15 min at 4°C and then anti-CD11b and anti-CD14 antibody, respectively, and analyzed by flow cytometry with FlowJo V10 software.

Results: As shown in FIG. 8, the induced differentiated DC cells were CD11b⁺ CD14⁻, indicating successful induction of differentiation.

### Example 12: Mixed lymphocyte response (MLR) evaluation of the effect of AD4-12 on T cell activation

CD4⁺ T cells were isolated from pre-differentiated DC cells (see Example 11) resuspended in RPMI1640 medium (containing 10% FBS) using a CD4 T cell negative selection kit (Miltenyi, Cat# 130-096-533). 1.4×10⁶ cells /mL of CD4⁺ T cells and 1.4×10⁵ cells/mL of pre-differentiated DC cells were mixed at a volume ratio of 1.1, and incubated with 150 µL/well of DC:T (1:10) cell mixture and then 10 µg/mL antibody and isotype for 2 days at 37 °C in a 5% CO₂ incubator. IL2 concentration was quantified using an IL-2 assay kit (R&D Systems, Cat#D2050) after 2 days. IFNγ concentration was quantified using a kit (R&D Systems, Cat# DIF50C) after another 3 days.

Results: As shown in FIG. 9, in the presence of PD-L1 antibody avelumab (Ave), T cells were activated to release IL-2 and IFNγ at increased levels. The release of IL-2 and IFNγ by T cells did not reduce after the addition of AD4-12-G1 or AD4-12-G1ₗₐₗₐ compared to isotype, indicating AD4-12-G1 or AD4-12-G1ₗₐₗₐ do not affect the release of cytokines by T cell activation.

### Example 13: FACS detection of AD4-12 endocytosis

1) Antibody labeling:
   Antibodies were labeled with pHrodo (Thermo, Cat# Z25611), which shows fluorescence only when entering lysosomes with low intracellular pH, to detect its endocytosis on a flow cytometer. 160 nM of antibodies diluted in CD fusion medium or isotype was mixed with a 12.5-fold dilution of pHrodo master at a volume ratio of 1:1 and incubated for 10 min at 37°C in an incubator.
2) Detection of antibodies endocytosis
   293T-SIRPα V1 cells in the logarithmic phase were dispensed in a 96-well plate in CD fusion at a density of 1×10⁵ per well, incubated with 50 µL of antibody-pHrodo for 20 min on ice and then sampled as samples at 0 hr. After culturing in a 5% CO₂ incubator at 37°C, the remaining cells were sampled after 1 hr, 2 hrs, 4 hrs, 6 hrs, and 8 hrs in sequence. All samples were stored on ice and analyzed by flow cytometry with FlowJo V10 and GraphPad Prism6 software.

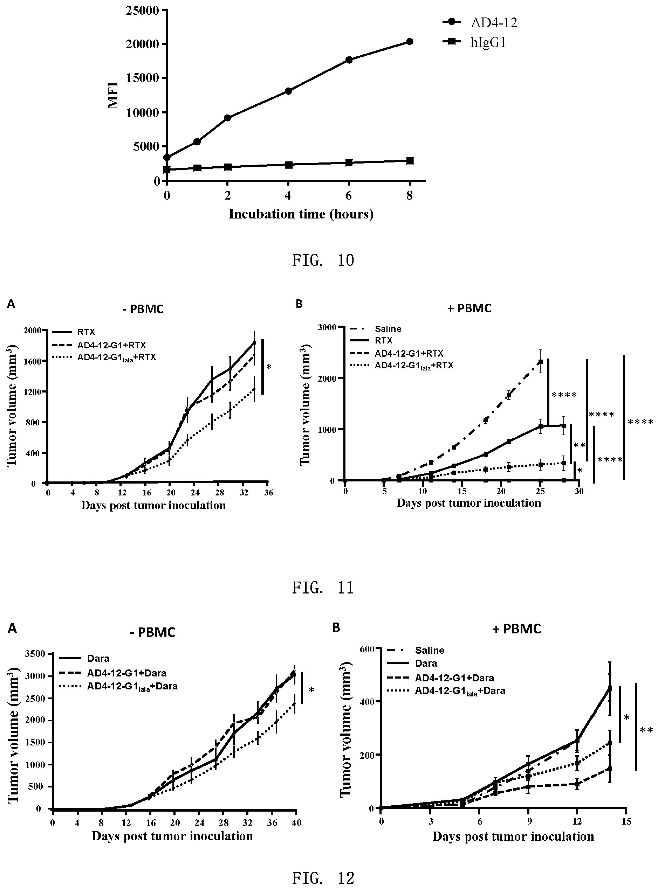

Results: As shown in FIG. 10, AD4-12 increased the fluorescence signal value as the incubation time of the cells at 37°C increased, indicating significant endocytosis.

### Example 14: Anti-tumor activity of AD4-12 in combination with rituximab

A. Raji cells in the logarithmic phase were resuspended in RPMI-1640 medium (containing 10% FBS and 1% sodium pyruvate). SIRPα-humanized complete immunodeficient mice (hSIRPα-B-NDG) (Biocytogen) were subcutaneously inoculated with 5×10⁵ Raji cells. Tumor volumes were measured twice weekly with vernier calipers.

Mice were divided into 3 groups of 7 mice each and intrathecally administered with 150 µg of rituximab alone or in combination with 200 µg of AD4-12-G1 or AD4-12-G1ₗₐₗₐ twice a week for a total of 6 doses when the tumors grew to a volume of ~100 mm³.

Results: As shown in FIG. 11A, when lacking T cells, B cells, and NK cells, with only myeloid cells remaining as immune effector cells, tumor growth did not differ between the group administered AD4-12-G1 in combination with rituximab and rituximab alone, while AD4-12-G1ₗₐₗₐ enhanced the tumor growth inhibitory effect of rituximab (*P < 0.05).

B. 2×10⁷/mL Raji cells in logarithmic phase were mixed with 1×10⁷/mL PBMC (Milestone^{®} Biotechnologies, Cat# PB003F-W) at a volume ratio of 1:1, and then Matrigel at a volume ratio of 1:1. Immunodeficient B-NDG mice (Biocytogen) were subcutaneously inoculated with 100 µL of the cells-Matrigel mixture. Tumor volumes were measured twice weekly with vernier calipers.

Each group of 5 mice was intraperitoneally administered saline, 20 µg of rituximab alone or in combination with 200 µg of AD4-12-G1 or AD4-12-G1ₗₐₗₐ once a week for a total of 3 doses, starting on the day of inoculation.

Results: As shown in FIG. 11B, when PBMC provided immune effector cells (including T cells, B cells, NK cells and myeloid cells), a low dose of rituximab significantly slowed tumor growth compared to saline, and either AD4-12-G1ₗₐₗₐ or AD4-12-G1 significantly enhanced the efficacy of rituximab (**P < 0.01, ****P < 0.0001), while the enhancement of AD4-12-G1 was more potent than that of AD4-12-G1ₗₐₗₐ (*P < 0.05).

### Example 15: Anti-tumor activity of AD4-12 in combination with daratumumab

A. hSIRPα-B-NDG were subcutaneously inoculated with 5×10⁵ Raji cells in the logarithmic phase. Tumor volumes were measured twice weekly with vernier calipers.

Mice were divided into 3 groups of 7 mice each and intrathecally administered with 150 µg of daratumumab alone or in combination with 200 µg of AD4-12-G1 or AD4-12-G1ₗₐₗₐ twice a week for a total of 6 doses when the tumors grew to a volume of ~100 mm³.

Results: As shown in FIG. 12A, when lacking T cells, B cells, and NK cells, with only myeloid cells remaining as immune effector cells, tumor growth did not differ between the group administered AD4-12-G1 in combination with daratumumab and daratumumab alone, while AD4-12-G1ₗₐₗₐ enhanced the tumor growth inhibitory effect of daratumumab (*P < 0.05).

B. 2×10⁷/mL Raji cells in logarithmic phase were mixed with 2×10⁷/mL PBMC (Milestone^{®} Biotechnologies, Cat# PB003F-W) at a volume ratio of 1:1, and then Matrigel at a volume ratio of 1:1. Immunodeficient B-NDG mice (Biocytogen) were subcutaneously inoculated with 100 µL of the cells-Matrigel mixture. Tumor volumes were measured twice weekly with vernier calipers.

Each group of 5 mice was intraperitoneally administered saline, 15 µg of daratumumab alone or in combination with 200 µg of AD4-12-G1 or AD4-12-G1ₗₐₗₐ once a week for a total of 3 doses, starting on the day of inoculation.

Results: As shown in FIG. 12B, when PBMC provided immune effector cells (including T cells, B cells, NK cells, and myeloid cells), a low dose of daratumumab did not slow tumor growth, whereas either AD4-12-G1ₗₐₗₐ or AD4-12-G1 significantly enhanced the efficacy of daratumumab (*P<0.05, **P<0.01).

### Example 16: Humanization of AD4-12

Comparing the AD4-12 sequence with the human immunoglobulin sequence library IMGT (Lefranc, 2003), the human germline sequence with the least amino acid sequence differences from the VH and VL framework regions of AD4-12 was selected as the template for humanization. The V and J germlines with the most similar AD4-12 sequence matches are shown in TABLE 4.

**TABLE 4. The V and J germline with the most similar AD4-12 sequence matches**

| Sequence | V germline | J germline |
|---|---|---|
| Heavy chain of AD4-12 | V4-30-4*01 | J6*01 |
| Light chain of AD4-12 | V7-3*01 | J4 |

First, the CDR region of AD4-12 was directly replaced with the CDR region of the humanized template to form heavy chain variable region VH (SEQ ID NO: 15) and light chain variable region VL (SEQ ID NO: 19) of the variant Z0. Second, VH and VL framework sequences were reverently mutated for sites that may be essential for maintaining the CDR conformation, respectively, and combined in pairs with VH and VL carrying different mutations to form the humanized variants Z0-Z9 (TABLE 5-6). Third, VL and VH were linked to the human κ light chain and IgG1 heavy chain constant region, respectively, with the Fc segment of the heavy chain constant region containing L234A/L235A(lala) mutation (SEQ ID NO: 13).

**TABLE 5. Humanized sequences and revertant mutations of AD4-12**

| ID | VH | SEQ ID NO: | VL | SEQ ID NO: |
|---|---|---|---|---|
| Z0 | GH | 15 | GL | 19 |
| Z1 | GH+F78V+S79F | 16 | GL+L50P+T87A | 20 |
| Z2 | GH+F78V+S79F | 16 | GL+G72R+T87A | 21 |
| Z3 | GH+F78V+S79F | 16 | GL+L50P+G72R+T87A | 22 |
| Z4 | GH+V71K+F78V+S79F | 17 | GL+L50P+T87A | 20 |
| Z5 | GH+V71K+F78V+S79F | 17 | GL+G72R+T87A | 21 |
| Z6 | GH+V71K+F78V+S79F | 17 | GL+L50P+G72R+T87A | 22 |
| Z7 | GH+V71K+F78V+S79F+A88D+V92R | 18 | GL+L50P+T87A | 20 |
| Z8 | GH+V71K+F78V+S79F+A88D+V92R | 18 | GL+G72R+T87A | 21 |
| Z9 | GH+V71K+F78V+S79F+A88D+V92R | 18 | GL+L50P+G72R+T87A | 22 |

**TABLE 6. Humanized sequences of AD4-12**

| Name | Amino acid sequence |
|---|---|
| IgG1 Fc L234A/L235A (lala) muatant (SEQ ID NO: 13) | EPKSCDKTHTCPPCPAPEAAGGPSVFLFPPKPKDTL MISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSR DELTKNQVSLTCLVKGFYPSDIAVEWESNGQPENN YKTTPPVLDSDGSFFLYSKLTVDKSRWQQGNVFSC SVMHEALHNHYTQKSLSLSPGK |
| Heavy chain variable domain of Z0 (SEQ ID NO: 15) | QVQLQESGPGLVKPSQTLSLTCTVSGFSLTGYGVN WIRQPPGKGLEWIGIIWGDGSTDYNSALKSRVTIS VDTSKNQFSLKLSSVTAADTAVYYCARAGKMDY WGQGTTVTVSS |
| Heavy chain variable domain of Z1 (SEQ ID NO: 16) | QVQLQESGPGLVKPSQTLSLTCTVSGFSLTGYGVN WIRQPPGKGLEWIGIIWGDGSTDYNSALKSRVTIS VDTSKNQVFLKLSSVTAADTAVYYCARAGKMDY WGQGTTVTVSS |
| Heavy chain variable domain of Z5 (SEQ ID NO: 17) | QVQLQESGPGLVKPSQTLSLTCTVSGFSLTGYGVN WIRQPPGKGLEWIGIIWGDGSTDYNSALKSRVTIS KDTSKNQVFLKLSSVTAADTAVYYCARAGKMDY WGQGTTVTVSS |
| Heavy chain variable domain of Z9 (SEQ ID NO: 18) | QVQLQESGPGLVKPSQTLSLTCTVSGFSLTGYGVN WIRQPPGKGLEWIGIIWGDGSTDYNSALKSRVTIS KDTSKNQVFLKLSSVTADDTARYYCARAGKMDY WGQGTTVTVSS |
| Light chain variable domain of Z0 (SEQ ID NO: 19) | DIVLTQSPASLAVSPGQRATITCRASESVDSYGNSF MHWYQQKPGQPPKLLIYLASNLESGVPARFSGSGS GTDFTLTINPVEANDTANYYCQQNNEYPWTFGGG TKVEIK |
| Light chain variable domain of Z1 (SEQ ID NO: 20) | DIVLTQSPASLAVSPGQRATITCRASESVDSYGNSF MHWYQQKPGQPPKPLIYLASNLESGVPARFSGSGS GTDFTLTINPVEANDAANYYCQQNNEYPWTFGGG TKVEIK |
| Light chain variable domain of Z5 (SEQ ID NO: 21) | DIVLTQSPASLAVSPGQRATITCRASESVDSYGNSF MHWYQQKPGQPPKLLIYLASNLESGVPARFSGSGS RTDFTLTINPVEANDAANYYCQQNNEYPWTFGGG TKVEIK |
| Light chain variable domain of Z9 (SEQ ID NO: 22) | DIVLTQSPASLAVSPGQRATITCRASESVDSYGNSF MHWYQQKPGQPPKPLIYLASNLESGVPARFSGSGS RTDFTLTINPVEANDAANYYCQQNNEYPWTFGGG TKVEIK |

### Example 17: Affinity detection of AD4-12 humanized variants by surface plasmon resonance (SPR)

Using the Biacore analysis system, activators were prepared by mixing 400 mM EDC and 100 mM NHS and activated with a CM5 sensor chip at a flow rate of 10 µL/min for 420 s. Anti-Fc antibody at 30 µg/mL dissolved in 10 mM NaAc (pH 4.5) was injected into the channel at a flow rate of 10 µL/min and then closed with 1 M ethanolamine-HCl at a flow rate of 10 µL/min for 420 s. Various humanized variants of AD4-12 were bound to the CM5 sensor chip at a flow rate of 10 µL/min, followed by the injection of 50 nM SIRPα V1-his (ACRO, Cat# SIA-H5225) into channels 1-8 at a flow rate of 30 µL/min for 120 s, followed by 300 s separation, after each dissociation phase with the injection of pH 1.5 of 10 mM glycine as regeneration buffer. All experimental data were fitted using a 1:1 binding model.

Results: As shown in TABLE 7, the affinity of humanized variants Z1, Z3-Z9 remained the same as that of AD4-12, and Z4 was selected as the humanized sequence of antibody AD4-12.

**TABLE 7. Affinity binding of AD4-12 humanized variants to SIRPα-V1 by SPR assay**

| ID | kₐ (1/Ms) | k_{d} (1/s) | K_{D} (M) |
|---|---|---|---|
| AD4-12 | 7.70E+05 | 1.13E-03 | 1.47E-09 |
| Z0 | fast on, fast off | | |
| Z1 | 7.44E+05 | 2.69E-03 | 3.61E-09 |
| Z2 | fast on, fast off | | |
| Z3 | 6.99E+05 | 2.92E-03 | 4.17E-09 |
| Z4 | 7.75E+05 | 1.54E-03 | 1.98E-09 |
| Z5 | 7.47E+05 | 2.08E-02 | 2.78E-08 |
| Z6 | 7.08E+05 | 1.65E-03 | 2.34E-09 |
| Z7 | 7.54E+05 | 1.51E-03 | 2.01E-09 |
| Z8 | 7.68E+05 | 1.94E-02 | 2.53E-08 |
| Z9 | 7.08E+05 | 1.60E-03 | 2.27E-09 |

### Example 18: Attempts to remove high-risk sites for post-translational modification (PTM) in CDR sequences

High risk sites for possible isomerization or deamidation were identified in the CDR sequence of the Z4 antibody, with one deamidation site (NS) and one isomerization site (DG) located on heavy chain CDR2 (SEQ ID NO: 6) and one deamidation site (NS) located on light chain CDR1 (SEQ ID NO: 7). To reduce the risk of PTM, we performed amino acid substitutions for these three potential PTM sites in the Z4 antibody sequence (TABLE 8). Two sites can be mutated for heavy chain CDR2, and the general formula can be summarized as IIWGDX₁STDYNX₂ALKS, where X₁ is G or A and X₂ is S or A, yielding an amino acid sequence consistent with that shown in SEQ ID NO: 6, SEQ ID NO: 14, SEQ ID NO: 23 or SEQ ID NO: 24. One site mutation can be performed for light chain CDR1, and the general formula can be summarized as RASESVDSYGX₃SFM, where X₃ is N or S, yielding an amino acid sequence consistent with that shown in SEQ ID NO: 8 or SEQ ID NO: 25.

**TABLE 8. PTM sites on CDR for amino acid substitution**

| Name | Amino acid sequence |
|---|---|
| HCDR2 (SEQ ID NO: 6) | IIWGDGSTDYNSALKS |
| HCDR2 (SEQ ID NO: 14) | IIWGDASTDYNAALKS |
| HCDR2 (SEQ ID NO: 23) | IIWGDASTDYNSALKS |
| HCDR2 (SEQ ID NO: 24) | IIWGDGSTDYNAALKS |
| LCDR1 (SEQ ID NO: 8) | RASESVDSYGNSFM |
| LCDR1 (SEQ ID NO: 25) | RASESVDSYGSSFM |

The mutated sequences were linked to the constant region of the human κ light chain and IgG1 heavy chain containing the L234A/L235A(lala) mutation, respectively, and their binding ability to SIRP family members was compared by flow cytometry. H7L4, in which both the NS site and DG site of heavy chain CDR2 were eliminated, was finally selected as our antibody drug candidate sequence (TABLE 9-11).

**TABLE 9. Amino acid substitution of PTM high prevalence sites in CDR sequences to form Z4 variants**

| ID | SEQ ID NO: | HCDR2 | SEQ ID NO: | LCDR1 | SEQ ID NO: |
|---|---|---|---|---|---|
| H4L3 | Heavy chain: 28 | VH | 6 | VL+N34S | 25 |
| | Light chain: 26 | | | | |
| H5L3 | Heavy chain: 29 | VH+G55A | 23 | VL+N34S | 25 |
| | Light chain: 26 | | | | |
| H6L3 | Heavy chain: 30 | VH+S61A | 24 | VL+N34S | 25 |
| | Light chain: 26 | | | | |
| H7L3 | Heavy chain: 31 | VH+G55A+S61A | 14 | VL+N34S | 25 |
| | Light chain: 26 | | | | |
| H4L4 | Heavy chain: 28 | VH | 6 | VL | 8 |
| | Light chain: 27 | | | | |
| H5L4 | Heavy chain: 29 | VH+G55A | 23 | VL | 8 |
| | Light chain: 27 | | | | |
| H6L4 | Heavy chain: 30 | VH+S61A | 24 | VL | 8 |
| | Light chain: 27 | | | | |
| H7L4 | Heavy chain: 31 | VH+G55A+S61A | 14 | VL | 8 |
| | Light chain: 27 | | | | |

**TABLE 10. CDR sequences of the H7L4 variable region**

| | CDR | SEQ ID NO: | Sequence |
|---|---|---|---|
| Heavy chain variable domain of H7L4 (SEQ ID NO: 11) | HCDR1 | 5 | GFSLTGYGVN |
| | HCDR2 | 14 | IIWGDASTDYNAALKS |
| | HCDR3 | 7 | AGKMDY |
| Light chain variable domain of H7L4 (SEQ ID NO: 12) | LCDR1 | 8 | RASESVDSYGNSFM |
| | LCDR2 | 9 | LASNLES |
| | LCDR3 | 10 | QQNNEYPWT |

**TABLE 11. Variable region sequences of the Z4 variant**

| Name | Amino acid sequence |
|---|---|
| Heavy chain variable domain of H7L4 < SEQ ID NO : 11) | QVQLQESGPGLVKPSQTLSLTCTVSGFSLTGYGVN WIRQPPGKGLEWIGIIWGDASTDYNAALKSRVTIS KDTSKNQVFLKLSSVTAADTAVYYCARAGKMDY WGQGTTVTVSS |
| Light chain variable domain of H7L4 < SEQ ID NO : 12) | DIVLTQSPASLAVSPGQRATITCRASESVDSYGNSFMHWY QQKPGQPPKPLIYLASNLESGVPARFSGSGSGTDFTLTINP VEANDAANYYCQQNNEYPWTFGGGTKVEIK |
| Light chain of H5L3 (SEQ ID NO: 26) | DIVLTQSPASLAVSPGQRATITCRASESVDSYGSSFMHWYQQ KPGQPPKPLIYLASNLESGVPARFSGSGSGTDFTLTINPVEAN DAANYYCQQNNEYPWTFGGGTKVEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSS PVTKSFNRGEC |
| Light chain of H7L4 (SEQ ID NO: 27) | DIVLTQSPASLAVSPGQRATITCRASESVDSYGNSFMHWYQ QKPGQPPKPLIYLASNLESGVPARFSGSGSGTDFTLTINPVEA NDAANYYCQQNNEYPWTFGGGTKVEIKRTVAAPSVFIFPPS DEQLKSGTASVVCLLNNFYPREAKVQWKVDNALQSGNSQ ESVTEQDSKDSTYSLSSTLTLSKADYEKHKVYACEVTHQGL SSPVTKSFNRGEC |
| Heavy chain of H4L3 (SEQ ID NO: 28) | QVQLQESGPGLVKPSQTLSLTCTVSGFSLTGYGVNWIRQPP GKGLEWIGIIWGDGSTDYNSALKSRVTISKDTSKNQVFLKL SSVTAADTAVYYCARAGKMDYWGQGTTVTVSSASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPEAAGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |
| Heavy chain of H5L3 < SEQ ID NO : 29) | QVQLQESGPGLVKPSQTLSLTCTVSGFSLTGYGVNWIRQPP GKGLEWIGIIWGDASTDYNSALKSRVTISKDTSKNQVFLKL SSVTAADTAVYYCARAGKMDYWGQGTTVTVSSASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPEAAGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |
| Heavy chain of H6L3 (SEQ ID NO: 30) | QVQLQESGPGLVKPSQTLSLTCTVSGFSLTGYGVNWIRQPP GKGLEWIGIIWGDGSTDYNAALKSRVTISKDTSKNQVFLKL SSVTAADTAVYYCARAGKMDYWGQGTTVTVSSASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPEAAGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |
| Heavy chain of H7L4 (SEQ ID NO: 31) | QVQLQESGPGLVKPSQTLSLTCTVSGFSLTGYGVNWIRQPP GKGLEWIGIIWGDASTDYNAALKSRVTISKDTSKNQVFLKL SSVTAADTAVYYCARAGKMDYWGQGTTVTVSSASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPEAAGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |

### Example 19: Construction of H7L4 with different Fc isoforms

The VL and VH fragment cDNAs of H7L4 were linked to human κ light chain and wild type IgG1 (SEQ ID NO: 32), IgG2 (SEQ ID NO: 33), IgG4 (SEQ ID NO: 34), and IgG1 heavy chain constant region containing the L234A/L235A (lala) mutation, respectively, to construct antibodies with different effector functional activity isoforms (TABLE 12). The light and heavy chains of H7L4 were cloned into the pCDNA3.1(+) expression vector to obtain H7L4-G1 (SEQ ID NO: 35, SEQ ID NO: 27), H7L4-G2 (SEQ ID NO: 36, SEQ ID NO: 27), H7L4-G4 (SEQ ID NO: 37, SEQ ID NO: 27) and H7L4-G1ₗₐₗₐ (SEQ ID NO: 31, SEQ ID NO: 27). ExpiCHO-S cells were transiently transferred into by the plasmid, cultured, and purified as in Example 2 above.

Reference BI 765063 was synthesized according to the sequence published in OSE Immunotherapeutics' patent CN201780023581.2, and the Fc fragment is IgG4.

**TABLE 12. H7L4 with different Fc subtypes**

| Name | Amino acid sequence |
|---|---|
| IgG1w Fc (SEQ ID NO: 32) | |
| IgG2 Fc (SEQ ID NO: 33) | ASTKGPSVFPLAPCSRSTSESTAALGCLVKDYFPEPVTVSWN SGALTSGVHTFPAVLQSSGLYSLSSVVTVPSSNFGTQTYTCN VDHKPSNTKVDKTVERKCCVECPPCPAPPVAGPSVFLFPPK PKDTLMISRTPEVTCVVVDVSHEDPEVQFNWYVDGVEVHN AKTKPREEQFNSTFRVVSVLTVVHQDWLNGKEYKCKVSNK GLPAPIEKTISKTKGQPREPQVYTLPPSREEMTKNQVSLTCL VKGFYPSDIAVEWESNGQPENNYKTTPPMLDSDGSFFLYSK LTVDKSRWQQGNVFSCSVMHEALHNHYTQKSLSLSPGK |
| IgG4 Fc < SEQ ID NO: 34) | ASTKGPSVFPLAPCSRSTSESTAALGCLVKDYFPEPVTVSWN SGALTSGVHTFPAVLQSSGLYSLSSVVTVPSSSLGTKTYTCN VDHKPSNTKVDKRVESKYGPPCPPCPAPEFLGGPSVFLFPPK PKDTLMISRTPEVTCVVVDVSQEDPEVQFNWYVDGVEVHN AKTKPREEQFNSTYRVVSVLTVLHQDWLNGKEYKCKVSN KGLPSSIEKTISKAKGQPREPQVYTLPPSQEEMTKNQVSLTC LVKGFYPSDIAVEWESNGQPENNYKTTPPVLDSDGSFFLYS RLTVDKSRWQEGNVFSCSVMHEALHNHYTQKSLSLSLGK |
| Heavy chain of H7L4-G1w (SEQ ID NO:35) | QVQLQESGPGLVKPSQTLSLTCTVSGFSLTGYGVNWIRQPP GKGLEWIGIIWGDASTDYNAALKSRVTISKDTSKNQVFLKL SSVTAADTAVYYCARAGKMDYWGQGTTVTVSSASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |
| Heavy chain of H7L4-G2 (SEQ ID NO:36) | QVQLQESGPGLVKPSQTLSLTCTVSGFSLTGYGVNWIRQPP GKGLEWIGIIWGDASTDYNAALKSRVTISKDTSKNQVFLKL SSVTAADTAVYYCARAGKMDYWGQGTTVTVSSASTKGPS VFPLAPCSRSTSESTAALGCLVKDYFPEPVTVSWNSGALTSG VHTFPAVLQSSGLYSLSSVVTVPSSNFGTQTYTCNVDHKPS NTKVDKTVERKCCVECPPCPAPPVAGPSVFLFPPKPKDTLMI SRTPEVTCVVVDVSHEDPEVQFNWYVDGVEVHNAKTKPR EEQFNSTFRVVSVLTVVHQDWLNGKEYKCKVSNKGLPAPI EKTISKTKGQPREPQVYTLPPSREEMTKNQVSLTCLVKGFY PSDIAVEWESNGQPENNYKTTPPMLDSDGSFFLYSKLTVDK SRWQQGNVFSCSVMHEALHNHYTQKSLSLSPGK |
| Heavy chain of H7L4-G4 (SEQ ID NO:37) | QVQLQESGPGLVKPSQTLSLTCTVSGFSLTGYGVNWIRQPP GKGLEWIGIIWGDASTDYNAALKSRVTISKDTSKNQVFLKL SSVTAADTAVYYCARAGKMDYWGQGTTVTVSSASTKGPS VFPLAPCSRSTSESTAALGCLVKDYFPEPVTVSWNSGALTSG VHTFPAVLQSSGLYSLSSVVTVPSSSLGTKTYTCNVDHKPSN TKVDKRVESKYGPPCPPCPAPEFLGGPSVFLFPPKPKDTLMI SRTPEVTCVVVDVSQEDPEVQFNWYVDGVEVHNAKTKPR EEQFNSTYRVVSVLTVLHQDWLNGKEYKCKVSNKGLPSSIE KTISKAKGQPREPQVYTLPPSQEEMTKNQVSLTCLVKGFYP SDIAVEWESNGQPENNYKTTPPVLDSDGSFFLYSRLTVDKSR WQEGNVFSCSVMHEALHNHYTQKSLSLSLGK |

### Example 20: Comparison of the binding ability of antibodies before and after humanization (AD4-12 vs. H7L4) to SIRPα -V1, V2, V8 by FACS

2×10⁵ per well of stable transfer construct cell lines 293T-SIRPα-V1 (SEQ ID NO: 2), CHOZN-SIRPα-V2 (SEQ ID NO: 38), and CHOZN-SIRPα-V8 (SEQ ID NO: 39) in logarithmic phase were dispended in 96-well U-shaped culture plates, and incubated with Fc blocker and then 2 µg/mL antibodies for 30 min at 4°C. After washing, the cells were incubated with 0.2 µg of goat anti-hIgG-AF488 for 30 min at 4°C, then resuspended in 50µL of DPBS/1%FBS and analyzed by flow cytometry with FlowJo V10 and GraphPad Prism6 software.

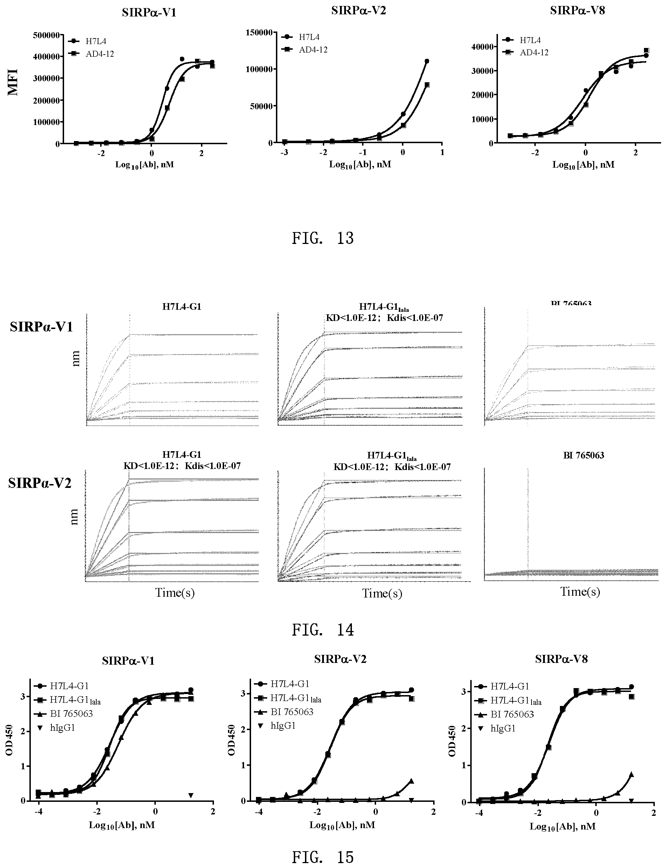

RESULTS: As shown in FIG. 13, there was no difference in the binding of the murine parent antibody AD4-12 and the humanized antibody H7L4 to SIRPα-V1, SIRPα-V2, and SIRPα-V8.

**TABLE 13. Sequences of SIRPα-V2 and SIRPα-V8**

| Name | Amino acid sequence |
|---|---|
| SIRPα-V2 (SEQ ID NO: 38) | MEPAGPAPGRLGPLLCLLLAASCAWSGVAGEEELQVIQPDK SVSVAAGESAILHCTVTSLIPVGPIQWFRGAGPARELIYNQK EGHFPRVTTVSESTKRENMDFSISISNITPADAGTYYCVKFR KGSPDTEFKSGAGTELSVRAKPSAPVVSGPAARATPQHTVS FTCESHGFSPRDITLKWFKNGNELSDFQTNVDPVGESVSYSI HSTAKVVLTREDVHSQVICEVAHVTLQGDPLRGTANLSETIR VPPTLEVTQQPVRAENQVNVTCQVRKFYPQRLQLTWLENG NVSRTETASTVTENKDGTYNWMSWLLVNVSAHRDDVKLT CQVEHDGQPAVSKSHDLKVSAHPKEQGSNTAAENTGSNER NIYIVVGVVCTLLVALLMAALYLVRIRQKKAQGSTSSTRLH EPEKNAREITQDTNDITYADLNLPKGKKPAPQAAEPNNHTE YASIQTSPQPASEDTLTYADLDMVHLNRTPKQPAPKPEPSFS EYASVQVPRK |
| SIRPα-V8 (SEQ ID NO: 39) | MEPAGPAPGRLGPLLCLLLAASCAWSGVAGEEELQVIQPDK SVLVAAGETATLRCTATSLIPVGPIQWFRGAGPARELIYNQK EGHFPRVTTVSESTKRENMDFSISISNITPADAGTYYCVKFR KGSPDTEFKSGAGTELSVRAKPSAPVVSGPAARATPQHTVS FTCESHGFSPRDITLKWFKNGNELSDFQTNVDPVGESVSYSI HSTAKVVLTREDVHSQVICEVAHVTLQGDPLRGTANLSETIR VPPTLEVTQQPVRAENQVNVTCQVRKFYPQRLQLTWLENG NVSRTETASTVTENKDGTYNWMSWLLVNVSAHRDDVKLT CQVEHDGQPAVSKSHDLKVSAHPKEQGSNTAAENTGSNER NIYIVVGVVCTLLVALLMAALYLVRIRQKKAQGSTSSTRLH EPEKNAREITQDTNDITYADLNLPKGKKPAPQAAEPNNHTE YASIQTSPQPASEDTLTYADLDMVHLNRTPKQPAPKPEPSFS EYASVQVPRK |

### Example 21: Affinity assay of H7L4 with SIRPα-V1, V2 by BLI

The affinity of H7L4 binding to SIRPα-V1, V2 was determined by ForteBio Octet RED 96. 5 µg/mL SIRPα V1-his and SIRPα V2-his was loaded to a Ni-NTA biosensor for 600 s, respectively. H7L4-G1, H7L4-G1ₗₐₗₐ, and BI 765063 were prepared at a 1:2 diluted series of 7 concentrations. The binding time was 200 s and the dissociation time was 600 s. The data were fitted to calculate the affinity.

Results: As shown in FIG. 14, both H7L4-G1 and H7L4-G1ₗₐₗₐ had high affinity for SIRPα-V1 and V2, while BI 765063 bound to SIRPα-V1 but not SIRPα-V2.

### Example 22: The binding of H7L4 to SIRPα -V1, V2, V8 detected by ELISA

100 µL of 1 µg/mL of SIRPα-V1-his, SIRPα-V2-his and SIRPα-V8-his (Sino Biological, Cat# 30015-H08H) were coated overnight at 4°C in Maxisorp ELISA 96-well plates. After blocking, serial dilutions of H7L4-G1, H7L4-G1ₗₐₗₐ, and reference BI 765063 were added to the plate and incubated for 2 hrs at 37°C. After washing, 100 uL of 1:5000 dilution of goat Anti-hIgG Fc HRP (Invitrogen, Cat# A18823) was added to each well and incubated for 1 h. TMB substrate was used for color development and enzyme marker readings.

Results: As shown in FIG. 15, H7L4-G1 and H7L4-G1ₗₐₗₐ bound well to SIRPα-V1, SIRPα-V2 and SIRPα-V8, while the reference BI 765063 bound SIRPα-V1 but not SIRPα-V2 and V8.

### Example 23: Affinity assay of H7L4 with SIRPα-V1, V2, V8, and SIRPβ, SIRPγ by FACS

2×10⁵ per well stable transferred cell lines 293T-SIRPα-V1 (SEQ ID NO: 2) cells, CHOZN-SIRPα-V2 (SEQ ID NO: 38) cells, 293T-SIRPα-V8 (SEQ ID NO: 39) cells, 293T-SIRPβ-V1 (Genbank Accession: 000241 sequence (30-371)) cells in logarithmic phase were dispensed into 96-well U-shaped culture plates, incubated with Fc blocker and then serially diluted of H7L4-G1, H7L4-G1ₗₐₗₐ and BI 765063 at 4°C for 30 min. After washing, cells were stained by 0.2 µg of goat anti-hIgG-AF488 at 4°C for 30 min, resuspended in 50 µL of DPBS/1% FBS, and analyzed by FlowJo V10 and GraphPad Prism6 software.

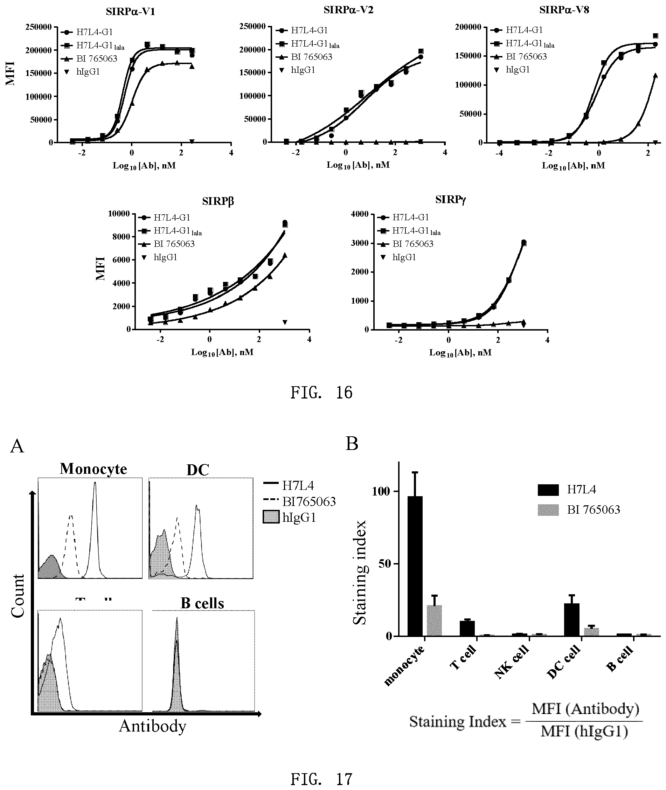

Results: As shown in FIG. 16, H7L4-G1 and H7L4-G1ₗₐₗₐ bound well to SIRPα-V1, SIRPα-V2, and SIRPα-V8 and relatively weakly to SIRPβ and SIRPγ, while BI 765063 bound to SIRPα-V1 and SIRPβ close to H7L4 and did not bind to SIRPα-V2 and SIRPγ at all.

### Example 24: Binding of H7L4 to human PBMC by FACS

5×10⁵/well of human PBMC (Milestone^{®} Biotechnologies, Cat# PB003F) were dispensed into 96-well U-shaped plates. After blocking with Fc blocker formulated in 1 mg/mL mIgG, cells were incubated with 3 µg of biotin-labeled H7L4-G1, BI 765063 or isotype control and then 0.2 µg of cell surface marker antibodies (including anti-CD56-APC, anti-CD11b- eFlour 506, anti-CD3-FITC, anti-CD14-APC eFlour 780 and anti-CD19-eFlour 450) and streptavidin-PE at 4°C for 30 min in turn. After washing, cells were resuspended in DPBS/1%FBS buffer containing 0.5 µg of 7AAD and analyzed by FACS.

Staining index = MFI (AD4-12)/MFI (isotype).

Results: As shown in FIG. 17, the binding of H7L4 or BI 765063 to each subpopulation of cells was ranked from strong to weak in order of monocytes (CD11b⁺CD14⁺), DC cells (CD11b⁺CD14⁻) and T cells (CD3⁺CD56⁻), without binding to B cells (CD19⁺CD3⁻) and NK cells (CD3⁻CD56⁺). The binding activity of H7L4 was seemed to higher than BI 765063 in all cases, which may due to the different efficiency of biotin labeling and the unknown SIRPα genotype of the donor.

### Example 25: Blockade of SIRPα-V1, V2 binding to CD47 by H7L4 detected by ELISA

100 µL of 1 µg/mL of recombinant SIRPα-V1-his and SIRPα-V2-his were coated overnight at 4°C in Maxisorp ELISA 96-well plates. Serially diluted H7L4-G1, H7L4-G1ₗₐₗₐ, and reference BI 765063 were mixed with 0.5 µg of biotin-CD47 and incubated in the microtiter plate for 1 h at 37°C. After washing, each well was added with 0.2 µg Streptavidin-HRP, substrate TMB, and read by an enzyme marker.

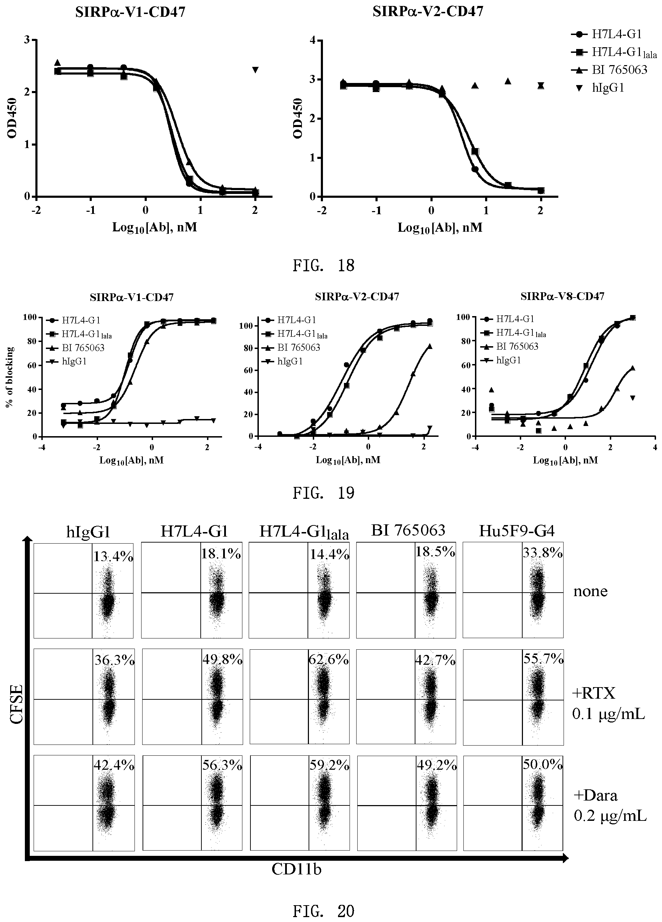

Results: As shown in FIG. 18, H7L4-G1 and H7L4-G1ₗₐₗₐ blocked the binding of SIRPα-V1 and V2 to CD47, and the reference BI 765063 showed comparable blocking activity to H7L4 for SIRPα-V1-CD47, but not for SIRPα-V2-CD47.

### Example 26: Blockade of SIRPα-V1, V2, V8 binding to CD47 by H7L4 detected by FACS

2×10⁵ cells/well logarithmically grown 293T-SIRPα-V1 cells, CHOZN-SIRPα-V2 cells, and 293T-SIRPα-V8 were dispensed into 96-well U-shaped culture plates, incubated with Fc blocker and then 0.5 µg of anti-CD47-FITC (homemade) and serially diluted H7L4-G1, H7L4-G1ₗₐₗₐ and BI 765063 at 4°C for 30 min. After washing, cells were analyzed by utilizing FlowJo V10 and GraphPad Prism6 software.

Results: As shown in FIG. 19, H7L4-G1 and H7L4-G1ₗₐₗₐ both blocked the binding of SIRPα-V1, V2, and V8 to CD47; while BI 765063 only blocked the binding of SIRPα-V1 to CD47, but not block SIRPα-V2 and V8.

### Example 27: Pro-phagocytosis of H7L4 in combination with Rituximab and Daratumumab

The experimental method refers to Example 10, and the antibodies used and the concentrations thereof were: 10 µg/mL of H7L4-G1, H7L4-G1ₗₐₗₐ or reference BI 765063, Hu5F9-G4 (CD47 antibody Magrolimab, sequence published according to WHO Drug Information, Vol. 33, No. 3, 2019 construct plasmid expression), and isotype control hIgG1; 0.1 µg/mL of rituximab (RTX) or 0.2 µg/mL of daratumumab (Dara).

Results: As shown in FIG. 20, no pro-phagocytic effect was observed with SIRPα antibody alone, and only CD47 antibody Hu5F9-G4 showed a significant pro-phagocytic effect. Either CD47 or SIRPα antibody in combination with RTX or Dara significantly increased antibody-dependent cellular phagocytosis (ADCP) of the latter, and the enhancement of ADCP of H7L4-G1ₗₐₗₐ was slightly better than that of H7L4-G1, BI 765063 or Hu5F9-G4.

### Example 28: ADCP effect of H7L4 on SIRPα⁺ cells detected by macrophage phagocytosis

The experimental method refers to Example 10, and the target cells were CFSE-labeled U937 cells (high expression of both SIRPα and CD47). The antibodies used and the concentrations thereof were: 10 µg/mL of H7L4-G1, H7L4-G1ₗₐₗₐ or reference BI 765063, Hu5F9-G4, and isotype control hIgG 1.

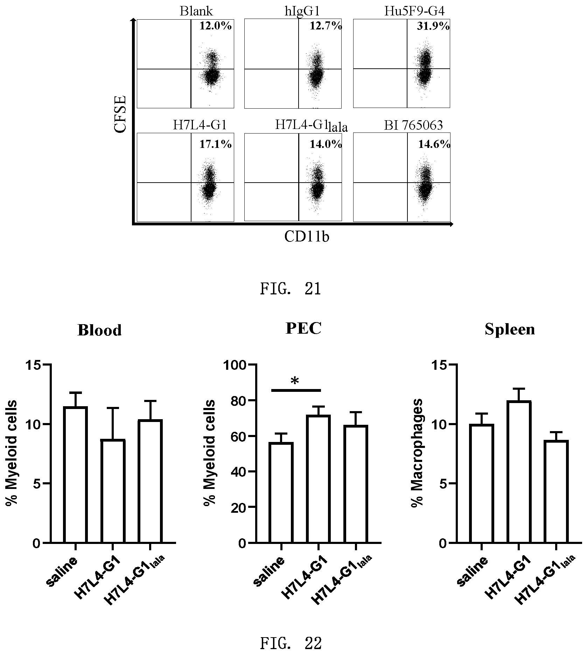

Results: As shown in FIG. 21, SIRPα antibodies including H7L4-G1, H7L4-G1ₗₐₗₐ and reference BI 765063 did not mediate ADCP in SIRPα-strongly positive U937 cells, while reference Hu5F9-G4 showed a significant ADCP effect.

### Example 29: The reduction of H7L4 on SIRPα⁺ cells in mice detected by FACS

SIRPα/CD47-double humanized C57BL/6 mice (hSIRPα-hCD47-B6, purchased from Biocytogen) were intraperitoneally injected with starch broth to induce macrophage exudation. 3 days later, 200 µg of H7L4-G1, H7L4-G1ₗₐₗₐ, or saline was injected intraperitoneally into three mice in each group. Another 3 days later, peripheral blood, spleen, and abdominal exudate were collected for flow cytometric analysis. 100 µL of peripheral blood was collected from each mouse, lysed by hypotonicity to remove red blood cells and transferred into a 96-well plate. Single cell suspension of the spleen was prepared by the mechanical grinding method, and 2×10⁵ cells per sample were transferred to a 96-well plate. The peritoneal exudate was centrifuged and resuspended, then 2×10⁵ cells from each sample were transferred to a 96-well plate. The cells in the 96-well plate were blocked with Fc blocker, added fluorescein-labeled anti-mouse CD11b antibody or F4/80 antibody, incubated at 4 °C for 30 min, washed, resuspended, flow cytometry topped, and analyzed.

Results: As shown in FIG. 22, H7L4-G1 slightly reduced CD11b⁺ myeloid cells in peripheral blood (not reaching a significant difference) but not F4/80⁺ macrophages in the spleen. CD11b⁺ myeloid cells in the peritoneal exudate increased rather than decreased (p<0.05).

### Example 30: MLR evaluation of the effect of H7L4 on T cell activation

CD4⁺ T cells were isolated from pre-differentiated DC cells (see Example 11) resuspended in RPMI1640 medium (containing 10% FBS) using a CD4 T cell negative selection kit (Miltenyi, Cat# 130-096-533). 1.4×10⁶ cells /mL of CD4⁺ T cells and 1.4×10⁵ cells/mL of pre-differentiated DC cells were mixed at a volume ratio of 1.1, and incubated with 150 µL/well of DC:T cell mixture and then 10 µg/mL H7L4-G1, BI 765063 and isotype for 2 days at 37 °C in a 5% CO₂ incubator. The supernatant was sampled to quantify IL2 concentration using an IL-2 assay kit. After replenishment (with samples of the same concentration), the plates were placed back in a 5% CO₂ incubator at 37 °C and continued to incubate for 3 days. After washing, T cell proliferation was detected by flow cytometry according to the principle that CFSE fluorescence was diluted with cell division and analyzed by FlowJo V10 software.

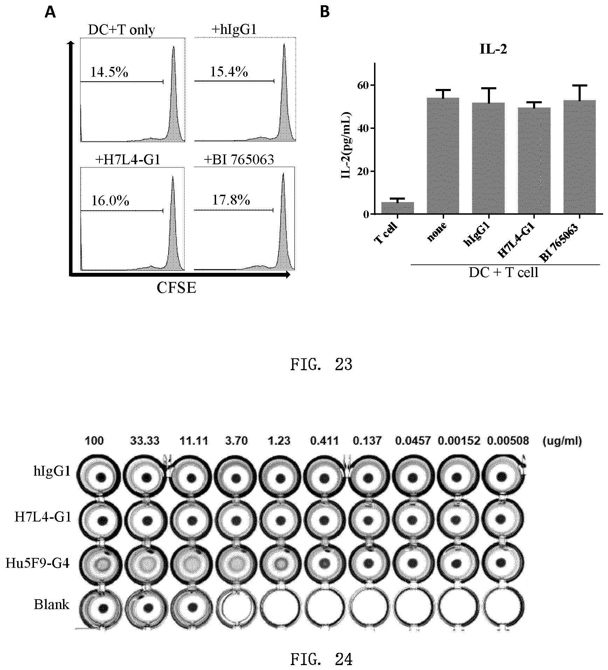

Results: As shown in FIG. 23, neither H7L4-G1 nor the reference BI 765063 altered T (A) cell proliferation and (B) activation (IL-2 release) compared to the blank control or IgG1 isotype control.

### Example 31: Red blood cell (RBC) agglutination assay

RBCs were resuspended in DPBS at a density of 6%(v/v). H7L4-G1, Hu5F9-G4, and hIgG1 were serially 3-fold diluted in DPBS at a maximum concentration of 100 µg/ml in 10 gradients. 50 µL/well of antibodies solution was added to a 96-well U-bottom plate, followed by 50 µL/well of the RBC suspension, incubated at 37 °C for 3 hrs, and then transferred to 4 °C for overnight incubation. The results were recorded using Chemi Doc XRS+ Imaging System.

Results: As shown in FIG. 24, Hu5F9-G4 significantly caused RBC agglutination, while H7L4-G1 did not cause RBC agglutination.

### Example 32: Cytokine release assay induced by H7L4 incubation with PBMC

Fresh PBMC (Milestone^{®} Biotechnologies, Cat# PB003F) from 3 donors were dispended in 3 replicate wells of U-bottom 96-well plates at a density of 0.5×10⁶ cells per well, and incubated for 48 hrs with H7L4-G1, H7L4-G1ₗₐₗₐ or reference BI 765063 at a final concentration of 10 µg/mL and a blank control without antibody. The supernatant was analyzed with 27 cytokines released mainly by macrophages (monocytes) and T cells using the multi-factor platform Luminex LXR-M500KCAF0Y by Unin (CRO).

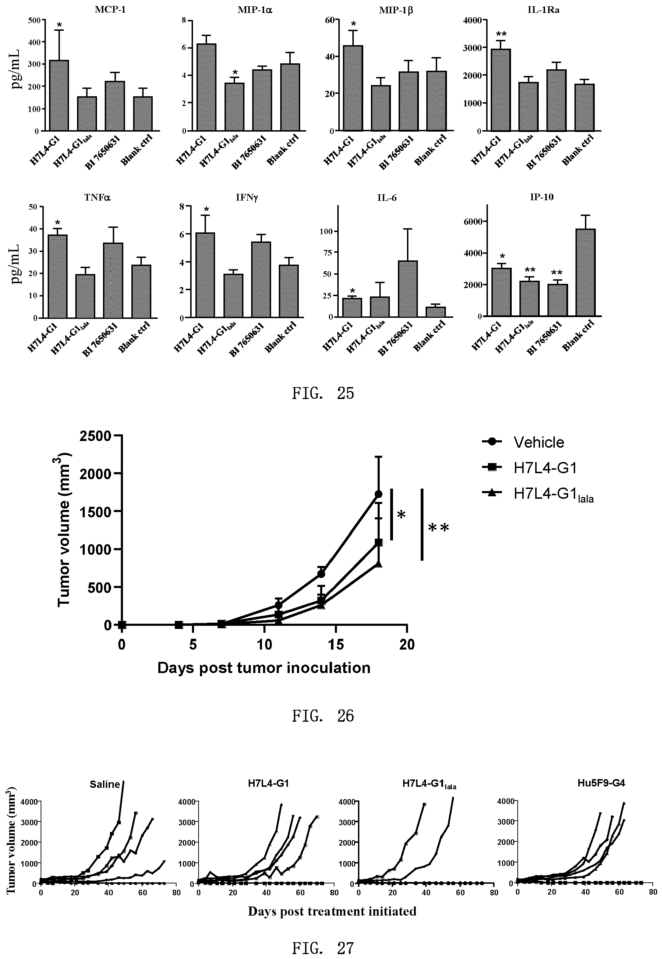

Results: As shown in FIG. 25, H7L4-G1 increased the release of MCP-1, MIP-1α, MIP-1β, IL-1Ra, TNFα, IFNγ, and IL-6 by PBMC, while decreased the release of IP-10. H7L4-G1ₗₐₗₐ decreased the release of MIP-1α and IP-10 secreted by PBMC. Other factors were below the limit of detection or did not differ from the blank control.

### Example 33: Anti-tumor activity of H7L4 in immunocompetent mice (A20 lymphoma cell line)

SIRPα/CD47-double humanized Balb/c (hSIRPα-hCD47-Balb/c, purchased from GemPharmatech, Jiangsu) were inoculated subcutaneously with 5×10⁵ human CD47-transferred A20 lymphoma cells with 6 mice per group. When the tumor volume reached 100-200 mm³, 200 µg of H7L4-G1 or H7L4-G1ₗₐₗₐ was administered intraperitoneally twice a week for a total of 4 doses. Tumor volumes were measured twice a week.

Results: As shown in FIG. 26, H7L4-G1 and H7L4-G1ₗₐₗₐ significantly inhibited the growth of tumors compared to saline.

### Example 34: Anti-tumor activity of H7L4 in immunocompetent mice (MC38 colon cancer cell line)

hSIRPα-hCD47-B6 (Biocytogen) were inoculated subcutaneously with 5×10⁵ human CD47-transferred MC38 colon cancer cells with 6 mice per group. When the tumor volume reached 100-200 mm³, 200 µg of H7L4-G1, H7L4-G1ₗₐₗₐ, or Hu5F9-G4 was administered intraperitoneally twice a week for a total of 6 doses. Tumor volumes were measured twice a week.

Results: As shown in FIG. 27, the tumor disappeared in 4/6 mice in the H7L4-G1ₗₐₗₐ-treated group and grew to the euthanasia endpoint in 4/6 mice in the other groups.

### Example 35: Anti-tumor activity of H7L4 in combination with a PD-L1 mAb in immunocompetent mice (MC38 colon cancer cell line)

hSIRPα-hCD47-B6 (Biocytogen) were inoculated subcutaneously with 1×10⁶ human CD47-transferred MC38 colon cancer cells with 6 mice per group. When the tumor volume reached 100-200 mm³, 200 µg of H7L4-G1, 100 µg of avelumab (a PD-L1 mAb), or both of them was administered intraperitoneally once every 3 days for a total of 3 doses. Tumor volumes were measured twice a week.

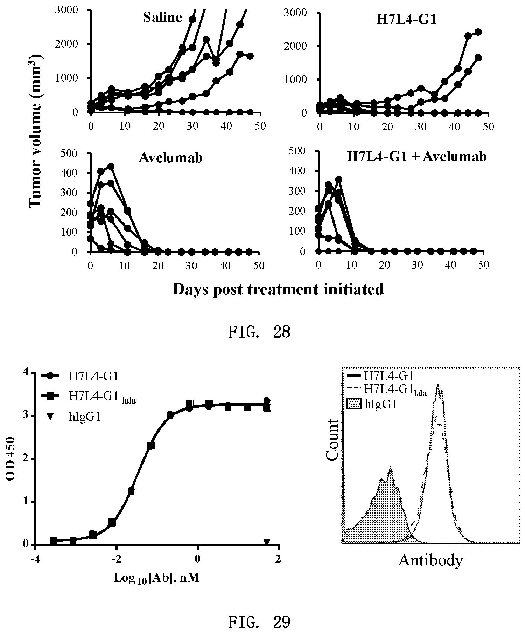

Results: As shown in FIG. 28, tumors disappeared in 4/6 mice in the H7L4-G1-treated group, and in all in the Avelumab-treated mice. Tumor regression was faster in the H7L4-G1 combined with the Avelumab-treated group, suggesting a synergistic effect.

### Example 36: Binding of H7L4 to cynomolgus macaques SIRPα by ELISA and FACS

### A. Binding of H7L4 to cynomolgus macaques SIRPα by ELISA

100 µL of 1 µg/mL of cynomolgus macaques SIRPα-his were coated overnight at 4°C in Maxisorp ELISA 96-well plates. Serially diluted H7L4-G1 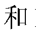 H7L4-G1ₗₐₗₐ was added to the plate for 1 h at 37°C. After washing, each well was incubated with 100 µL of 1:5000-fold diluted goat anti-hIgG Fc HRP for 1 h at 37°C and then substrate TMB, and read by an enzyme marker.

### B. Binding of H7L4 to cynomolgus macaques SIRPα by FACS

4×10⁵/well of cynomolgus macaques PBMC (a gift from Unin) were dispensed into 96-well U-shaped plates. After blocking with Fc blocker formulated in 1 mg/mL mIgG for 15 min at 4°C, cells were incubated with biotin-labeled H7L4-G1, H7L4-G1ₗₐₗₐ, and isotype control for 30 min at 4°C, and then 0.2 µg of Streptavidin-PE and anti-CD11b-FITC at 4°C for 30 min. After washing, cells were resuspended in DPBS/1%FBS buffer containing 0.5 µg of 7AAD and analyzed by flow cytometry.

Results: As shown in FIG. 29, H7L4-G1 and H7L4-G1ₗₐₗₐ bound cynomolgus macaques SIRPα well at both molecular and cell levels.

## Claims

1. A monoclonal antibody targeting SIRPα or an antigen-binding portion thereof, which comprises:
a) a heavy chain variable region CDR1 comprising the amino acid sequence set forth in SEQ ID NO: 5,
b) a heavy chain variable region CDR2 comprising the amino acid sequence set forth in IIWGX₁X₂STDYX₃X₄ALKS, wherein X₁ is D, E or N, X₂ is G, A or S, X₃ is N, Q or S, X₄ is S, T or A,
c) a heavy chain variable region CDR3 comprising the amino acid sequence set forth in SEQ ID NO: 7,
d) a light chain variable region CDR1 comprising the amino acid sequence set forth in RASESVDSYGX₅X₆FM, wherein X₅ is N, Q or S, X₆ is S, T or A,
e) a light chain variable region CDR2 comprising the amino acid sequence set forth in SEQ ID NO: 9, and
f) a light chain variable region CDR3 comprising the amino acid sequence set forth in SEQ ID NO: 10.

2. The monoclonal antibody or the antigen-binding portion thereof according to claim **1,** which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 6, SEQ ID NO: 14, SEQ ID NO: 23 or SEQ ID NO: 24,
c) a heavy chain variable region CDR3 comprising the amino acid sequence set forth in SEQ ID NO: 7,
d) a light chain variable region CDR1 comprising the amino acid sequence set forth in SEQ ID NO: 8 or SEQ ID NO: 25,
e) a light chain variable region CDR2 comprising the amino acid sequence set forth in SEQ ID NO: 9, and
f) a light chain variable region CDR3 comprising the amino acid sequence set forth in SEQ ID NO: 10.

3. The monoclonal antibody or the antigen-binding portion thereof according to claim **1,** which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 6,
c) a heavy chain variable region CDR3 comprising the amino acid sequence set forth in SEQ ID NO: 7,
d) a light chain variable region CDR1 comprising the amino acid sequence set forth in SEQ ID NO: 8,
e) a light chain variable region CDR2 comprising the amino acid sequence set forth in SEQ ID NO: 9, and
f) a light chain variable region CDR3 comprising the amino acid sequence set forth in SEQ ID NO: 10.

4. The monoclonal antibody or the antigen-binding portion thereof according to claim **1,** which comprises:
a) a heavy chain variable region CDR1 comprising the amino acid sequence set forth in SEQ ID NO: 5,
b) a heavy chain variable region CDR2 comprising the amino acid sequence set forth in SEQ ID NO: 14,
c) a heavy chain variable region CDR3 comprising the amino acid sequence set forth in SEQ ID NO: 7,
d) a light chain variable region CDR1 comprising the amino acid sequence set forth in SEQ ID NO: 8,
e) a light chain variable region CDR2 comprising the amino acid sequence set forth in SEQ ID NO: 9, and
f) a light chain variable region CDR3 comprising the amino acid sequence set forth in SEQ ID NO: 10.

5. The monoclonal antibody or the antigen-binding portion thereof according to any one of claims **1-4,** which is derived from the antibody having high homology to the CDR region or variable region of the monoclonal antibody targeting SIRPα by modifying the conserved sequence, including substitutions, additions, and deletions of one or more amino acids.

6. The monoclonal antibody or the antigen-binding portion thereof according to claim **5,** wherein the number of said additions, deletions, and/or substitutions of one or more amino acids do not exceed five.

7. The monoclonal antibody or the antigen-binding portion thereof according to claim **6,** wherein the number of said additions, deletions, and/or substitutions of one or more amino acids do not exceed three.

8. A monoclonal antibody targeting SIRPα or the antigen-binding portion thereof, which comprises:
a) a heavy chain variable region comprising the amino acid sequence set forth in SEQ ID NO: 3, SEQ ID NO: 16, SEQ ID NO: 17 or SEQ ID NO: 18,
b) a light chain variable region comprising the amino acid sequence set forth in SEQ ID NO: 4, SEQ ID NO: 20, SEQ ID NO: 21, or SEQ ID NO: 22.

9. The monoclonal antibody or the antigen-binding portion thereof according to claim **8,** wherein the heavy chain variable region thereof has at least 90%, 91%, 92%, 93%, 94%, 95%, 96%, 97%, 98%, 99% or 100% sequence homology with the amino acid sequence selected from SEQ ID NO: 3, SEQ ID NO: 16, SEQ ID NO: 17 or SEQ ID NO: 18; the light chain variable region thereof has at least 90%, 91%, 92%, 93%, 94%, 95%, 96%, 97%, 98%, 99% or 100% sequence homology with the amino acid sequence selected from SEQ ID NO: 4, SEQ ID NO: 20, SEQ ID NO: 21 or SEQ ID NO: 22.

10. The monoclonal antibody or the antigen-binding portion thereof according to claim **8,** wherein the heavy chain and the light chain variable region of the said antibody comprises the sequence set forth in SEQ ID NO: 3 and SEQ ID NO: 4, respectively.

11. The monoclonal antibody or the antigen-binding portion thereof according to claim **8,** wherein the antibody is a humanized antibody with the heavy chain and the light chain variable region sequences selected from the group consisting of:
1) a heavy chain variable region comprising the sequence of SEQ ID NO: 16, and a light chain variable region comprising the sequence of SEQ ID NO: 20,
2) a heavy chain variable region comprising the sequence of SEQ ID NO: 16, and a light chain variable region comprising the sequence of SEQ ID NO: 22,
3) a heavy chain variable region comprising the sequence of SEQ ID NO: 17, and a light chain variable region comprising the sequence of SEQ ID NO: 20,
4) a heavy chain variable region comprising the sequence of SEQ ID NO: 17, and a light chain variable region comprising the sequence of SEQ ID NO: 21,
5) a heavy chain variable region comprising the sequence of SEQ ID NO: 17, and a light chain variable region comprising the sequence of SEQ ID NO: 22,
6) a heavy chain variable region comprising the sequence of SEQ ID NO: 18, and a light chain variable region comprising the sequence of SEQ ID NO: 20,
7) a heavy chain variable region comprising the sequence of SEQ ID NO: 18, and a light chain variable region comprising the sequence of SEQ ID NO: 21,
8) a heavy chain variable region comprising the sequence of SEQ ID NO: 18, and a light chain variable region comprising the sequence of SEQ ID NO: 22.

12. The monoclonal antibody or the antigen-binding portion thereof according to claim **8,** wherein the antibody is a full-length antibody containing a constant region of human IgG

13. The monoclonal antibody or the antigen-binding portion thereof according to claim **8,** wherein the antibody is a full-length antibody containing a constant region of mouse IgG

14. The monoclonal antibody or the antigen-binding portion thereof according to claim **12,** wherein the antibody heavy chain constant region is selected from human IgG1, IgG2, IgG3, or IgG4 constant regions.

15. The monoclonal antibody or the antigen-binding portion thereof according to claim **14,** wherein the human constant region Fc structural domain is an IgG1, IgG2, or IgG4 Fc structural domain.

16. The monoclonal antibody or the antigen-binding portion thereof according to claim **15,** wherein the human constant region Fc structural domain is an IgG1 mutant (LALA) structural domain or an IgG1 wild type structural domain.

17. The monoclonal antibody or the antigen-binding portion thereof according to claim **12,** wherein the antibody light chain constant region is a κ or λ constant region, but preferably a κ constant region.

18. The monoclonal antibody or the antigen-binding portion thereof according to claim **8,** wherein the antibody or antibody fragment is a human antibody or a human antibody fragment.

19. The monoclonal antibody or the antigen-binding portion thereof according to claim **18,** wherein the antibody fragment is a Fab, Fab', Fab'-SH, Fv, scFv or F(ab')2 antibody fragment.

20. The monoclonal antibody or the antigen-binding portion thereof according to claim **18,** wherein the antibody fragment is bispecific.

21. A monoclonal antibody targeting SIRPα, which comprises:
a) a heavy chain comprising the amino acid sequence set forth in SEQ ID NO: 28, SEQ ID NO: 29, SEQ ID NO: 30, SEQ ID NO: 31, SEQ ID NO: 35, SEQ ID NO: 36 or SEQ ID NO: 37, and
b) a light chain comprising the amino acid sequence set forth in SEQ ID NO: 26 or SEQ ID NO: 27.

22. The monoclonal antibody according to claim **21,** wherein the heavy chain (HC) thereof has a homologous sequence of at least 91%, 92%, 93%, 94%, 95%, 96%, 97%, 98%, 99%, or 100% sequence identity with the amino acid sequence selected from SEQ ID NO: 28, SEQ ID NO: 29, SEQ ID NO: 30, SEQ ID NO: 31, SEQ ID NO: 35, SEQ ID NO: 36 or SEQ ID NO: 37; the light chain (LC) thereof has a homologous sequence of at least 91%, 92%, 93%, 94%, 95%, 96%, 97%, 98%, 99% or 100% sequence identity with the amino acid sequence selected from SEQ ID NO: 26 or SEQ ID NO: 27.

23. The monoclonal antibody according to claim **22,** wherein an antibody having 90% or higher homology to the heavy and light chains of the said antibody can be obtained by mutagenesis, with the preferred site-directed mutagenesis or PCR-mediated mutagenesis sites being located at sites other than the heavy chain variable region CDR1-CDR3 and the light chain variable region CDR1-CDR3.

24. A monoclonal antibody targeting SIRPα, which comprises:
a) a heavy chain comprising the amino acid sequence set forth in SEQ ID NO: 31, SEQ ID NO: 35, SEQ ID NO: 36 or SEQ ID NO: 37, and
b) a light chain comprising the amino acid sequence set forth in SEQ ID NO: 27.

25. The monoclonal antibody according to claim **21,** wherein the antibody has a heavy chain and a light chain which sequence selected from the following group:
1) a heavy chain comprising the sequence set forth in SEQ ID NO: 28, and a light chain comprising the sequence set forth in SEQ ID NO: 26,
2) a heavy chain comprising the sequence set forth in SEQ ID NO: 29, and a light chain comprising the sequence set forth in SEQ ID NO: 26,
3) a heavy chain comprising the sequence set forth in SEQ ID NO: 30, and a light chain comprising the sequence set forth in SEQ ID NO: 26,
4) a heavy chain comprising the sequence set forth in SEQ ID NO: 31, and a light chain comprising the sequence set forth in SEQ ID NO: 26,
5) a heavy chain comprising the sequence set forth in SEQ ID NO: 28, and a light chain comprising the sequence set forth in SEQ ID NO: 27,
6) a heavy chain comprising the sequence set forth in SEQ ID NO: 29, and a light chain comprising the sequence set forth in SEQ ID NO: 27,
7) a heavy chain comprising the sequence set forth in SEQ ID NO: 30, and a light chain comprising the sequence set forth in SEQ ID NO: 27,
8) a heavy chain comprising the sequence set forth in SEQ ID NO: 31, and a light chain comprising the sequence set forth in SEQ ID NO: 27,
9) a heavy chain comprising the sequence set forth in SEQ ID NO: 35, and a light chain comprising the sequence set forth in SEQ ID NO: 27,
10) a heavy chain comprising the sequence set forth in SEQ ID NO: 36, and a light chain comprising the sequence set forth in SEQ ID NO: 27,
11) a heavy chain comprising the sequence set forth in SEQ ID NO: 37, and a light chain comprising the sequence set forth in SEQ ID NO: 27.

26. A monoclonal antibody targeting SIRPα, which comprises:
a) a heavy chain comprising the amino acid sequence set forth in SEQ ID NO: 31, a light chain comprising the amino acid sequence set forth in SEQ ID NO: 27, or
b) a heavy chain comprising the amino acid sequence set forth in SEQ ID NO: 35, a light chain comprising the amino acid sequence set forth in SEQ ID NO: 27.

27. An nucleic acid molecule encoding the monoclonal antibody targeting SIRPα or antigen-binding portion thereof of any one of claims **1-26.**

28. The nucleic acid molecule according to claim **27,** which is DNA or RNA, and can contain or not contain an intron sequence.

29. The nucleic acid molecule according to claim **28,** which is a cDNA molecule.

30. An expression vector, which comprises the nucleic acid molecule as claimed in any one of claims **27-29.**

31. A host cell, which is obtained by transformation or transfection of a prokaryotic or eukaryotic host cell by the vector of claim **30.**

32. The host cell according to claim 31, which is a bacterial, yeast or mammalian cell.

33. The host cell according to claim **32,** which is a mammalian cell selected from Chinese hamster ovary cells (CHO cells), NSO myeloma cells, COS cells, and SP2 cells.

34. The host cell according to claim **33,** which is a CHO cell.

35. A pharmaceutical composition, which comprises the monoclonal antibody targeting SIRPα or antibody fragment thereof of any one of claims **1-26** and a pharmaceutically acceptable carrier.

36. A pharmaceutical composition, which comprises the monoclonal antibody targeting SIRPα or antibody fragment thereof of any one of claims **1-26,** Rituximab, and a pharmaceutically acceptable carrier.

37. A pharmaceutical composition, which comprises the monoclonal antibody targeting SIRPα or antibody fragment thereof of any one of claims **1-26,** Daratumumab, and a pharmaceutically acceptable carrier.

38. A pharmaceutical composition, which comprises the monoclonal antibody targeting SIRPα or antibody fragment thereof of any one of claims **1-26,** a PD-(L)1 antibody and a pharmaceutically acceptable carrier, wherein the PD-(L)1 antibody comprises Nivolumab, Pembrolizumab, Cemiplimab-rwlc, Camrelizumab, Sintilimab, Toripalimab, Tislelizumab, Zimberelimab, Penpulimab, Serplulimab, Pucotenlimab, Atezolizumab, durvalumab, Avelumab, Envafolimab, Sugemalimab and Cadonilimab.

39. The use of the monoclonal antibody targeting SIRPα or antibody fragment thereof of any one of claims **1-26** in the treatment of oncological disease or microbial infectious disease.

40. The use according to claim **39,** wherein the use is for the treatment of cancer or the inhibition of tumor growth.

41. The use according to claim **40,** wherein the tumor or cancer is selected from squamous cell carcinoma, small cell lung cancer, non-small cell lung cancer, squamous non-small cell lung cancer (NSCLC), non-squamous NSCLC, glioma, gastrointestinal cancer, kidney cancer, ovarian cancer, liver cancer, colorectal cancer, endometrial cancer, prostate cancer, thyroid cancer, neuroblastoma, pancreatic cancer, glioblastoma, cervical cancer, gastric cancer, bladder cancer, head and neck cancer, melanoma, bone cancer, skin cancer, diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, and any combination of these cancers.

42. The use of the monoclonal antibody targeting SIRPα or antibody fragment thereof in combination with Rituximab or Daratumumab in the treatment of oncological disease of any one of claims **1-26.**

43. The use according to claim **42,** wherein the oncological disease is a hematological tumor.

44. The use according to claim **43,** wherein the hematological neoplastic disease is diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, etc.

45. The use of the monoclonal antibody targeting SIRPα or antibody fragment thereof in combination with a PD-(L)1 antibody in the treatment of oncological disease as claimed in any one of claims **1-26.**

46. The use according to claim **45,** wherein the PD-(L)1 antibody comprises a PD-1 antibody, a PD-L1 antibody, or a bispecific antibody against PD-1/PD-L1.

47. The use according to claim **46,** wherein the PD-1 antibody comprises Nivolumab, Pembrolizumab, Cemiplimab-rwlc, Camrelizumab, Sintilimab, Toripalimab, Tislelizumab, Zimberelimab, Penpulimab, Serplulimab, Pucotenlimab, etc.; and said PD-L1 monoclonal antibody comprises Atezolizumab, durvalumab, Avelumab, Envafolimab, Sugemalimab, etc.; and said PD-L1 bispecific antibody comprises Cadonilimab targeting PD-1 and CTLA-4.

48. The use according to claim **45,** wherein the oncological disease is hematological tumors, malignant melanoma, breast cancer, small cell lung cancer, non-small cell lung cancer, liver cancer, gastric cancer, kidney cancer, colorectal cancer, bladder cancer, head and neck tumors, cervical cancer, Merkel cell carcinoma, and all microsatellite highly unstable (MSI-H) solid tumor treatments, etc.

49. The use according to claim **48,** wherein the hematological neoplastic disease is diffuse large B-cell lymphoma, non-Hodgkin's lymphoma, acute lymphoblastic leukemia (ALL), acute myeloid leukemia (AML), chronic lymphocytic leukemia (CLL), chronic myeloid leukemia (CML), multiple myeloma, etc.

50. The use according to claim **39,** wherein the use for the treatment of microbial infectious diseases is for the treatment of viral infections or bacterial infectious diseases.

51. The use according to claim **50,** wherein the use is for the treatment of viral infections or bacterial infectious diseases, wherein said viral infectious diseases include diseases caused by infections such as adenovirus, herpes virus, papillomavirus, coronavirus, human immunodeficiency virus (HIV), human cytomegalovirus, EB virus, hepatitis C virus or hepatitis B virus; said bacterial infectious diseases include diseases caused by infections such as Bacillus, Chlamydia pneumoniae, Haemophilus influenzae, Mycobacterium tuberculosis, Pseudomonas, Salmonella, Staphylococcus, Streptococcus, and dense spirochetes.

52. The use according to claim **39,** wherein a therapeutically effective amount of the monoclonal antibody targeting SIRPα or antibody fragment thereof as described in any of claims **1-26** is administered to a subject in need of treatment.

53. The use according to claim **52,** wherein the subject is a human or non-human animal.

54. The use according to claim **53,** wherein the non-human animal comprises non-human primates, sheep, dogs, mice, rats, cats, cattle, horses, chickens, amphibians, and reptiles.

55. The use according to claim **52,** wherein the antibody is administered at a dose in the range of 0.01 - 100 mg/kg.

56. The use according to claim **55,** wherein the antibody is administered at a dose in the range of 0.1 mg/kg - 100 mg/kg, or 0.5 mg/kg - 50 mg/kg, or 1 mg/kg - 25 mg/kg, or 2 mg/kg - 10 mg/kg, or 5 mg/kg - 10 mg/kg.

57. The use according to claim **52,** wherein the therapeutic regimen of the antibody requires weekly, biweekly, every three weeks, every four weeks, monthly, every 3 months, or every 3 - 6 months administration.
